# EUROPEAN PATENT APPLICATION

(11) **EP 2 573 192 A1**
(43) Date of publication of application: **27.03.2013**
(21) Application number: 11783657.7
(22) Date of filing: 20.05.2011
(51) Int. Cl.: C12Q 1/68, C07K 16/00, C12N 15/00, G01N 33/53

(54) **METHOD AND KIT FOR DIAGNOSING AIM-RELATED ILLNESS**

(30) Priority: 20.05.2010 US 346811 P
(71) Applicant: Miyazaki, Toru, Tokyo 112-0003 (JP); Dainippon Sumitomo Pharma Co., Ltd., Osaka-shi, Osaka 541-8524 (JP)
(72) Inventor: MIYAZAKI, Toru, Bunkyo-ku, Tokyo 112-0003 (JP)
(74) Representative: HOFFMANN EITLE
(86) International application number: PCT/JP2011/061657
(87) International publication number: WO 2011/145725

(57) **Abstract**

The present invention aims to provide a diagnostic or detection method of an AIM-related disease, which is highly sensitive, highly specific, convenient and less burdensome for patients. The present invention provides a diagnostic method for an AIM-related disease, which includes a step of measuring an AIM concentration in a sample collected from a subject.

## Description

### Technical Field

The present invention relates to a diagnostic method and a diagnostic kit for AIM-related diseases such as metabolic syndrome and the like.

### Background Art

Conventionally, it is known that lifestyle-related diseases such as diabetes, hyperlipidemia, hypertension and obesity often accumulate in one person. While these diseases individually become a risk of arteriosclerosis, when accumulated, the risk becomes very high. Therefore, there are plural names that have been proposed to express the pathology of accumulation of these diseases.
At present, a disease concept including diabetes, hyperlipidemia, hypertension, obesity and insulin resistance as basic constituent factors, wherein these diseases often accumulate in one person and cause a high risk of arteriosclerotic diseases, is expressed by a standardized name of metabolic syndrome.

Generally, in metabolic syndrome, visceral fat type obesity, namely, obesity wherein neutral fats are intraperitoneally accumulated in excess, is the core pathology. The insulin resistance, which is developed due to obesity, sequentially causes hypertension, diabetes, hyperlipidemia and the like, and the accumulation and linkage thereof cause arteriosclerotic diseases. This phenomenon is compared to the domino effect, and the concept of "metabolic domino" has been proposed in recent years.
That is, a series of pathologies progress one directionally, like domino effect which is difficult to bring back once it starts to topple, and various diseases are developed chronically in a sustained manner over a long term.

Therefore, the treatment of metabolic syndrome does not aim at mitigation of symptoms, but prevention of the onset of various diseases possibly developed in chain. Under the concept of metabolic domino, since the progression once made is difficult to bring back to the original state as mentioned above, prophylaxis needs to started in an early stage, for which a diagnostic method capable of evaluating the risk of metabolic syndrome in an early stage is needed.

Heretofore, various diagnostic criteria of metabolic syndrome have been proposed. Waist diameter, serum triglyceride, HDL cholesterol, blood pressure, fasting blood glucose level, impaired glucose tolerance, insulin resistance, urine albumin content and the like have been recited as diagnosis items. When some of these items are met, metabolic syndrome is diagnosed in practice.
However, there are a variety of discussions over the current diagnostic criteria of metabolic syndrome, and some reports show that each diagnostic item does not necessarily correlate to the risk of diseases related to metabolic syndrome. This is considered to be attributable to the diversity of pathology of metabolic syndrome, diagnostic items not based on the mechanism of the onset and progress of metabolic syndrome and the like.

In the meantime, the present inventors have found Apoptosis Inhibitor of Macrophage (AIM) from macrophages in a thymus (see non-patent document 1). AIM is a soluble protein, and is a member of the scavenger receptor cysteine-rich (SRCR) superfamily. AIM was first found as an apoptosis inhibitor that protects macrophage from various apoptosis inducers (see non-patent document 1).
Since the structure of AIM, which has 3 SRCR domains, is similar to the extracellular domain of CD5, it is also called CD5L (CD5-like).

### [Document List]

### [non-patent document]

non-patent document 1: Miyazaki, T. et al., J. Exp. Med. 189, 413-422 (1999)

### SUMMARY OF THE INVENTION

### Problems to be Solved by the Invention

The present invention aims to provide a diagnostic method of metabolic syndrome and the like, which is highly sensitive, highly specific, convenient and less burdensome for patients.

### [Means of Solving the Problems]

During further studies of AIM, the present inventors have found that AIM is directly involved in the onset of metabolic syndrome.
To be precise, it has already been known that macrophages infiltrate into adipose tissues and cause chronic inflammation of adipose tissues and the whole body, which induces insulin resistance and cause a series of disease linkage in metabolic syndrome. The present inventors have found that migration of macrophages into adipose tissues is caused by increased blood concentration of AIM due to obesity.
Therefore, the risk of linkage of a series of diseases in metabolic syndrome can be diagnosed earlier at the most upstream stage by detecting the increase of blood AIM concentration.
The present inventors have further found that the concentration of AIM in the body fluid and tissues of a subject reflects not only metabolic syndrome but also risk, progression, prognosis and the like of various diseases whose onset and suppression involve AIM, which resulted in the completion of the present invention.
Accordingly, the present invention relates to
[1] A method for the diagnosis or detection of an AIM-related disease, comprising
   a step of measuring an AIM concentration in a sample collected from a subject,
   a step of comparing the aforementioned AIM concentration and an AIM concentration in a sample of a healthy subject;
[2] the method of the above-mentioned [1], wherein the aforementioned sample is a body fluid, an organ or a tissue;
[3] the method of the above-mentioned [2], wherein the aforementioned body fluid is blood;
[4] the method of the above-mentioned [2], wherein the aforementioned tissue is an adipose tissue;
[5] the method of any one of the above-mentioned [1] to [4], wherein the aforementioned step of measuring an AIM concentration is performed by a method selected from the group consisting of an immunoassay, an agglutination method, turbidimetry, a Western blotting method and a surface plasmon resonance method;
[6] the method of the above-mentioned [5], wherein the aforementioned immunoassay is selected from the group consisting of enzyme immunoassay (EIA or ELISA), radio immunoassay (RIA), fluorescence immunoassay (FIA), fluorescence polarization immunoassay (FPIA) and chemical luminescence immunoassay (CLIA), each using an anti-AIM antibody;
[7] the method of any one of the above-mentioned [1] to [6], wherein the aforementioned AIM-related disease is at least one disease selected from the group consisting of metabolic syndrome or a related disease thereof, cancer, infectious disease, degenerative disease of the brain and chronic inflammatory disease;
[8] the method of the above-mentioned [7], wherein the aforementioned AIM-related disease is metabolic syndrome or a related disease thereof, and
   when, in the aforementioned step of comparing the AIM concentration and the AIM concentration in a sample of a healthy subject, the AIM concentration in the sample of the subject is significantly higher than that in the sample of the healthy subject, the subject is judged to have been affected or have a high risk of being affected with metabolic syndrome or a related disease thereof;
[9] the method of the above-mentioned [8], wherein the aforementioned metabolic syndrome or a related disease thereof is at least one selected from the group consisting of metabolic syndrome, obesity, insulin resistance, diabetes, hyperlipemia, hypertension, arteriosclerotic disease, cerebrovascular disorder, ischemic heart disease, heart failure, dementia, cerebral apoplexy, neurosis, renal disease, abnormal secretion of adipocytokine and abnormal amount of blood free fatty acid;
[10] the method of any one of the above-mentioned [1] to [9], wherein the subject is a human;
[11] the method of any one of the above-mentioned [1] to [9], wherein the aforementioned subject is a non-human mammal or bird;
[12] the method of the above-mentioned [11], wherein the aforementioned subject is a dog or cat;
[13] a diagnostic or detection kit for an AIM-related disease, comprising an anti-AIM antibody;
[14] the kit of the above-mentioned [13], wherein the aforementioned AIM-related disease is at least one selected from the group consisting of metabolic syndrome, cancer, infectious disease, degenerative disease of the brain and chronic inflammatory disease;
[15] the kit of the above-mentioned [14], wherein the aforementioned metabolic syndrome or related diseases thereof is at least one selected from the group consisting of metabolic syndrome, obesity, insulin resistance, diabetes, hyperlipidemia, hypertension, arteriosclerotic disease, cerebrovascular disorder, ischemic heart disease, heart failure, dementia, cerebral apoplexy, neurosis, renal disease, secretion abnormality of adipocytokine, and abnormal amount of blood free fatty acid;
[16] the kit of any one of the above-mentioned [13] to [15], wherein the aforementioned anti-AIM antibody is a monoclonal antibody;
[17] a method of using an anti-AIM antibody, which is for the diagnosis or detection of an AIM-related disease;
[18] a method of using an anti-AIM antibody, which is for the production of a diagnostic reagent or a test reagent for an AIM-related disease;
[19] the method of the above-mentioned [17] or [18], wherein the aforementioned anti-AIM antibody is a monoclonal antibody;
[20] a method of using AIM or an AIM fragment, which is for the production of an anti-AIM antibody for a diagnostic reagent or a test reagent for an AIM-related disease;
[21] the method of the above-mentioned [20], wherein the aforementioned AIM fragment is selected from fragments containing a functional domain and a conserved region of the AIM protein;
[22] a method of using CD36, which is for the diagnosis or detection of an AIM-related disease; and
[23] a method of using CD36, which is for the production of a diagnostic drug or a test drug for an AIM-related disease.

### Effect of the Invention

According to the diagnostic method for AIM-related diseases of the present invention, the risk, progression, prognosis and the like of AIM-related diseases can be judged by a convenient method of measuring the AIM concentration of a sample collected from a subject.
Particularly, since AIM is deeply involved in the cause of metabolic syndrome, the diagnostic method of the present invention is useful as a method for evaluating the risk of metabolic syndrome in an early stage. Since this method utilizes an index based on the onset mechanism of metabolic syndrome, it has high sensitivity and high specificity.
In addition, using the diagnostic kit for AIM-related diseases of the present invention, the diagnostic method for AIM-related diseases of the present invention can be conveniently performed.

### [Brief Description of the Drawings]

[Fig. 1] Fig. 1 shows the results of blood AIM concentration measurements of obese mice and normal mice.
[Fig. 2] Fig. 2 shows the results of detection, using anti-macrophage monoclonal antibody (F4/80) and the like, of macrophage infiltration in visceral fat tissues of obese AIM^{+/+} mice and obese AIM^{-/-} mice.
[Fig. 3] Fig. 3 shows the results of detection, using anti-macrophage monoclonal antibody (F4/80) and the like, of macrophage infiltration in visceral fat tissues at 3 weeks after systemic administration of rAIM to AIM^{-/-} mice.
[Fig. 4] Fig. 4 shows the measurement results of macrophage migration capability.
[Fig. 5] Fig. 5 are photographs showing the anatomy results of AIM^{+/+} mice and AIM^{-/-} mice after loading HFD for 12 weeks.
[Fig. 6] Fig. 6 shows the results of body weight and total fat amount of AIM^{+/+} mice and AIM^{-/-} mice after loading HFD for 12 weeks.
[Fig. 7] Fig. 7 shows the results of a glucose tolerance test performed on AIM^{+/+} mice and AIM^{-/-} mice before HFD loading.
[Fig. 8] Fig. 8 shows the results of a glucose tolerance test performed on AIM^{+/+} mice and AIM^{-/-} mice after HFD loading.
[Fig. 9] Fig. 9 shows the results of an insulin tolerance test performed on AIM^{+/+} mice and AIM^{-/-} mice before HFD loading.
[Fig. 10] Fig. 10 shows the results of an insulin tolerance test performed on AIM^{+/+} mice and AIM^{-/-} mice after HFD loading.
[Fig. 11] Fig. 11 shows the results of an insulin sensitivity test performed on AIM^{+/+} mice and AIM^{-/-} mice after HFD loading.
[Fig. 12] Fig. 12 shows the results of staining of sections produced from the visceral fat tissues of normal mice (mice without high-fat diet loading) and obese mice with anti-macrophage monoclonal antibody, anti-mouse AIM polyclonal antibody and anti-IL-6 antibody.
[Fig. 13] Fig. 13 shows the schedule of rAIM loading during culture of 3T3-L1 cell.
[Fig. 14] Fig. 14 shows the results of the cells stained with oil-red-O on Day 12 in the schedule shown in Fig. 13.
[Fig. 15] Fig. 15 shows the results of expression of adipocyte markers as measured by quantitative real-time PCR on day 12 in the schedule shown in Fig. 13.
[Fig. 16] Fig. 16A shows the results of mature adipocytes loaded with rAIM and stained with oil-red-O. Fig. 16B shows the size of lipid droplet and Fig. 16C shows the number of lipid droplet-containing cells per unit area.
[fig. 17] Fig. 17 shows the measurement results obtained by loading mature adipocytes with rAIM, and measuring glycerol and free fatty acid in the culture supernatant.
[Fig. 18] Fig. 18 shows the measurement results obtained by loading mature adipocytes with rAIM, and measuring the expression of lipid droplet formation-related genes by quantitative real-time PCR.
[Fig. 19] Fig. 19 shows the results of HE staining of adipose tissue section of AIM⁺/⁺ mice and AIM^{-/-} mice after administration of HFD for 20 days.
[Fig. 20] Fig. 20A shows the results obtained by loading differentiated or undifferentiated 3T3-L1 cells with rAIM, and staining the cells for AIM, PPARγ2 and DAPI. Fig. 20B shows the measurement results obtained by classifying the cells according to the expression level of PPARγ2 based on the results of Fig. 20A, and counting the number of rAIM-containing cells per 100 of each cell. Fig. 20C shows the results obtained by loading 3T3-L1 cells with rAIM, and staining the cells for AIM and endosome, or AIM and lysosome.
[Fig. 21] Fig. 21 shows the results of electron microscopic observation of a sample same as that of Fig. 20 after labeling AIM with gold nanoparticles.
[Fig. 22] Fig. 22 shows the results obtained by treating 3T3-L1 cells with CD36 neutralizing antibody and examining the effect of rAIM for endocytosis.
[Fig. 23] Fig. 23 shows the results obtained by intravenously injecting rAIM to CD36^{+/+} mice and CD36^{-/-} mice, and staining sections prepared from the adipose tissue for AIM and macrophage.
[Fig. 24] Fig. 24 shows the results obtained by directly administering rAIM labeled with HA-tag by injection to adipose tissue of AIM^{-/-} mice, performing co-immunoprecipitation by anti-HA antibody using the adipose tissue, and detecting FAS in the precipitate by Western blotting.
[Fig. 25] Fig. 25 shows the results obtained by confirming the binding of rAIM labeled with HA-tag and FAS labeled with FLAG-tag by co-immunoprecipitation using an anti-Flag antibody or an anti-HA antibody in HEK 293T cells.
[Fig. 26] Fig. 26 shows the results obtained by labeling each domain of FAS with Flag-tag, expressing same in HEK 293T cell that stably expresses AIM-HA, and confirming the binding of FAS and AIM by co-immunoprecipitation using an anti-Flag antibody or an anti-HA antibody.
[Fig. 27] Fig. 27 shows the measurement results of FAS activity of 3T3-L1 cells treated in the presence or absence of rAIM (5 µg/mL), and in the presence of C75 (25 µM), for 6 days.
[Fig. 28] Fig. 28 shows the measurement results of FAS activity in adipose tissue of AIM^{+/+} mice and AIM^{-/-} mice.
[Fig. 29] Fig. 29 shows the measurement results of FAS activity in adipose tissue of AIM^{-/-} mice administered with rAIM or BSA 3 hours before by topical injection into adipose.

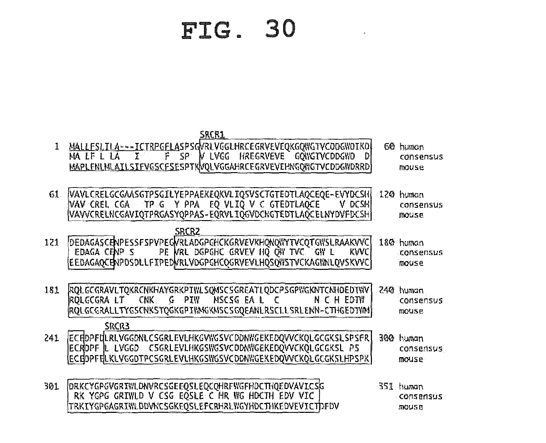

[Fig. 30] Fig. 30 shows amino acid sequences between human AIM and mouse AIM and the consensus.
[Fig. 31] Fig. 31 is a conceptual figure showing the structure of FAS.
[fig. 32] Fig. 32 shows the measurement results of the changes in visceral fat amount and subcutaneous fat amount of AIM^{+/+} mouse and AIM^{-/-} mice administered with HFD for 12 weeks.
[Fig. 33] Fig. 33 shows the measurement results of the changes in the body weight of AIM^{-/-} mice for 5 weeks, during which the mice were administered with HFD along with rAIM or BSA twice a week.
[Fig. 34] Fig. 34 shows the measurement results of the changes in the visceral fat amount and subcutaneous fat amount of AIM^{-/-} mice for 5 weeks, during which the mice were administered with HFD along with rAIM or BSA twice a week.
[Fig. 35] Fig. 35 shows the measurement results of the mRNA levels of adipocyte marker and the like in the visceral fat of AIM^{-/-} mice after the experiments shown in Figs. 33 and 34.
[Fig. 36] Fig. 36 shows the detection results of AIM protein in the sera of dog, cat and mouse by Western blotting.
[Fig. 37] Fig. 37 shows the measurement results of blood AIM concentration of about 550 patients who underwent comprehensive medical examination.
[Fig. 38] Fig. 38 shows the measurement results of blood AIM concentration of blood donors (including foreigners) having BMI of 18 - 25 or not less than 35, who were selected at random.

### Description of Embodiments

### [Diagnostic method of AIM-related disease]

The diagnostic or detection method for AIM-related disease of the present invention includes a step of measuring the AIM concentration in a sample collected from a subject.

In the present specification, "AIM" is, as mentioned above, a soluble protein, is a member of the SRCR superfamily and is known to express in human, many mammals other than human and birds. As one embodiment, the amino acid sequence of human AIM is shown in SEQ ID NO: 1. Those of ordinary skill in the art can evaluate whether or not a protein is a homologous protein of AIM in other mammals or birds, from the high level of sequence similarity and functional analysis. For example, the homology of amino acid sequences between mouse AIM and human AIM is as high as about 80%. In addition, the consensus sequence in the SRCR domain (amino acid sequence conserved in other molecules having an SRCR domain such as CD5, CD6 and the like) is completely the same between mouse AIM and human AIM.
In the present specification, AIM includes its analogs and variants. Examples of the analog and variant of AIM include a protein having an amino acid sequence of AIM wherein one to several amino acids have been deleted, substituted or added, and retaining the function of AIM. Those of ordinary skill in the art can determine whether a certain protein is an analog or a variant of AIM by the high level of sequence similarity or functional analysis.

In the present specification, AIM-related disease means any disease related to the function AIM exhibits in the body, and includes both the diseases AIM promotes their onset and progression, and the diseases suppressed or prevented by AIM.
The function AIM exhibits in the body here includes a function to suppress activity of fatty acid synthase (FAS) and any function AIM directly or indirectly exhibits. Examples of the function AIM directly or indirectly exhibits include a function causing binding to CD36 on an adipocyte surface; a function causing incorporation into adipocytes by endocytosis; a function causing binding to FAS in adipocytes; a function causing suppression of enzyme activity of FAS; a function causing promotion of lipid droplet fusion; a function inducing macrophage migration by lipid droplet fusion; a function to suppress apoptosis of macrophage and the like.
Therefore, examples of the disease related to the function AIM exhibits in the body include metabolic syndrome and related diseases thereof, cancer, infectious diseases (including mycobacterium infections such as tuberculosis and the like), degenerative diseases of the brain (including Alzheimer's disease) and chronic inflammatory diseases (autoimmune diseases). The concentration of AIM in a sample collected from a subject reflects the risk of the onset, progression, prognosis and the like of these diseases, and is understood to be useful for diagnoses.

### (Metabolic syndrome)

In the present specification, the metabolic syndrome is a concept showing a series of disease chain generally starting from visceral fat-type obesity (accumulation of visceral fat), then evoking insulin resistance via chronic inflammation of adipose tissue, secretion abnormality of adipocytokine from adipocyte, abnormal amount of blood free fatty acid and the like, thereafter inducing lifestyle-related diseases such as diabetes, hyperlipidemia, hypertension and the like, and finally developing various arteriosclerotic diseases. The downstream of the disease chain may include cerebrovascular disorder, ischemic heart diseases, heart failure, dementia, cerebral apoplexy, neurosis, renal diseases and the like.
In the present specification, the metabolic syndrome and related diseases include any diseases, symptoms and abnormalities based on various abnormalities produced in the mechanism of the onset or progression of metabolic syndrome, and in the process of the onset or progression of metabolic syndrome. For example, they include, but are not limited to, metabolic syndrome, obesity, insulin resistance, diabetes, hyperlipidemia, hypertension, arteriosclerotic diseases, cerebrovascular disorders, ischemic heart diseases, heart failure, dementia, cerebral apoplexy, neurosis, renal diseases, abnormal secretion of adipocytokine, and abnormal amount of blood free fatty acid.
The present inventors have found that, as shown in the below-mentioned Reference Examples, blood AIM concentration increases due to visceral fat-type obesity; as a result, AIM binds to CD36 expressed on the adipocyte surface and incorporated into adipocytes by endocytosis; AIM binds to fatty acid synthase (FAS) in adipocytes and suppresses its activity; which promotes lipid droplet fusion in adipocytes and induces macrophage migration; as a result, macrophage infiltrates into the adipose tissues to cause chronic inflammation in the adipose tissues and the whole body, whereby insulin resistance is induced.
Therefore, metabolic syndrome can be said an AIM-related disease, and the risk of metabolic syndrome and related diseases thereof can be evaluated at an early stage by detecting an increase in the AIM concentration in a sample collected from a subject.

As shown in the below-mentioned Examples, the blood AIM concentration was within the range of 5 - 20 µg/ml in many of the healthy individuals. The subjects with BMI of 35 or more showed a significantly higher blood AIM concentration as compared to those with BMI of 18 - 25.

### (Cancer)

Based on the function of AIM to suppress FAS activity, it is understood that cancer can also be diagnosed or detected by measuring the AIM concentration in the sample of a subject.
Examples of the cancer include colorectal cancer, breast cancer, lung cancer, prostate cancer, esophagus cancer, gastric cancer, liver cancer, biliary cancer, spleen cancer, kidney cancer, urinary bladder cancer, uterine cancer, ovarian cancer, testis cancer, thyroid cancer, pancreatic cancer, brain tumor, blood tumor and the like. Of these, the diagnostic method of the present invention is suitable for the diagnosis of breast cancer, prostate cancer and liver cancer.

### (Infectious disease)

Based on the function of AIM to suppress FAS activity, it is understood that infectious diseases can also be diagnosed or detected by measuring the AIM concentration in the sample of a subject.
While the kind of the infectious diseases is not particularly limited, for example, mycobacterium infections such as tuberculosis and the like can be mentioned.

### (Degenerative disease of the brain)

Based on the function of AIM to suppress FAS activity, it is understood that degenerative diseases of the brain can also be diagnosed or detected by measuring the AIM concentration in the sample of a subject.
While the kind of the degenerative diseases of the brain is not particularly limited, for example, Alzheimer's disease can be mentioned.

### (Chronic inflammatory disease)

Based on the function of AIM to suppress FAS activity, it is understood that chronic inflammatory diseases can also be diagnosed or detected by measuring the AIM concentration in the sample of a subject.
While the kind of the chronic inflammatory diseases is not particularly limited, for example, autoimmune disease can be mentioned.

In the present specification, the sample is not particularly limited as long as it can be collected from a subject, for example, a body fluid or a tissue can be used. Examples of the body fluid include, but are not limited to, blood, lymph fluid, tissue fluid, celomic fluid, digestive juice, snivel and urine. Examples of the tissue include, but are not limited to, adipose tissue.
Of these, blood is preferable from the aspects of collection and treatment.
As the blood sample, one collected from a subject and treated according to a conventional method can be used.

In AIM-related diseases, the AIM concentration in the sample of a subject shows a significant difference from that in the corresponding sample of a healthy subject. That is, when the AIM concentration in the sample of a healthy subject shows a significant difference from that in the sample of a subject, said subject is judged to have a high possibility of being affected with an AIM-related disease, or to be highly possibly affected with an AIM-related disease in the future.

A step of measuring the AIM concentration in a sample can be performed by any method used for the detection and measurement of a protein. Examples of such method include, but are not limited to, immunoassay, agglutination method, nephelometry, Western blotting method, surface plasmon resonance (SPR) method and the like.
Of these, an immunoassay utilizing an anti-AIM antibody and an antigen antibody reaction with AIM is particularly convenient and preferable, and the anti-AIM antibody is preferably a monoclonal antibody.

For the immunoassay, a detectably labeled anti-AIM antibody or a detectably labeled antibody (secondary antibody) to anti-AIM antibody is used. They are classified into enzyme immunoassay (EIA or ELISA), radio immunoassay (RIA), fluorescence immunoassay (FIA), fluorescence polarization immunoassay (FPIA), chemical luminescence immunoassay (CLIA) and the like according to the labeling method of an antibody, and any of these can be used for the method of the present invention.
For ELISA method, an enzyme such as peroxidase, alkaline phosphatase and the like is used; for RIA method, a radioactive substance such as ¹²⁵I, ¹³¹I, ³⁵S, ³H and the like is used; for FPIA method, a fluorescent substance such as fluorescein isothiocyanate, rhodamine, dansyl chloride, phycoerythrin, tetramethyl rhodamine isothiocyanate, near infrared fluorescence material and the like is used; and for CLIA method, an antibody labeled with a luminescence substance such as luciferase, luciferin, aequorin and the like is used. Besides these, an antibody labeled with a nanoparticle such as gold colloid, quantum dot and the like can also be detected.
In immunoassay, moreover, an anti-AIM antibody may be labeled with biotin, avidin or streptavidin labeled with an enzyme and the like is bound thereto, and AIM can be detected and measured.

Among immunoassays, ELISA method using enzyme labeling is preferable since an antigen can be measured conveniently and rapidly.
As the ELISA method, for example, sandwich method can be used. An anti-AIM antibody is immobilized on a solid phase carrier, a blood sample treated appropriately is added and reacted, and an anti-AIM antibody recognizing another epitope labeled with an enzyme is added and allowed to react.
After washing, the sample is reacted with an enzyme substrate to induce color development, and the absorbance is measured to determine AIM concentration. In addition, an anti-AIM antibody immobilized on a solid phase carrier may be reacted with AIM in a blood sample, an unlabeled anti-AIM antibody (primary antibody) is added, and an enzyme-labeled antibody (secondary antibody) to said unlabeled antibody may be further added.
When the enzyme is peroxidase, 3,3'-diaminobenzidine (DAB), 3,3' 5,5'-tetramethylbenzidine (TMB), o-phenylenediamine (OPD) and the like can be used as the enzyme substrate and, when it is alkali phosphatase, p-nitropheny phosphate (NPP) and the like can be used.

In the above-mentioned immunoassay, an agglutination method is also preferable as a method capable of conveniently detecting a trace amount of a protein. As the agglutination method, for example, latex agglutination wherein an antibody is bound with latex particles can be mentioned.
When latex particles bound with an anti-AIM antibody are mixed with the blood sample, in which AIM is present, the latex particles bound with the antibody coagulate. Then, a near infrared light is irradiated to the sample, aggregate mass is quantified by measuring the absorbance (turbidimetry) or scattering light (nephelometry), and the concentration of the antigen can be determined.

An anti-AIM antibody, both monoclonal antibody and polyclonal antibody, can be produced according to a known method. For example, a monoclonal antibody can be obtained by isolating antibody-producing cells from a non-human mammal immunized with AIM or AIM fragment, fusing this with myeloma cell and the like to produce a hybridoma and purifying the antibody produced by the hybridoma. In addition, a polyclonal antibody can be obtained from the serum of an animal immunized with AIM or AIM fragment.
AIM fragment is a partial peptide of AIM, and an anti-AIM antibody fragment recognizes AIM.

In the present specification, the "antibody" refers to a full-length immunoglobulin molecule or an immunologically active fragment of an immunoglobulin molecule such as antibody fragment, which is naturally occurring or produced by a genetic recombination technique. Unless otherwise specified, the antibody of the present specification include any type, class and subclass, for example, IgG, IgE, IgM, IgD, IgA, IgY, IgG1, IgG2, IgG3, IgG4, IgA1 and IgA2 and the like. These antibodies can be produced using a conventional technique, and may be a polyclonal antibody or a monoclonal antibody. Examples of the antibody fragment include F(ab')₂, F(ab)₂, Fab', Fab, Fv, scFv and the like. An antibody fragment can be produced by, for example, a genetic recombination technology and using a nucleic acid encoding same, or by cleaving a full-length antibody with an enzyme.

### [Detection method]

The present invention also encompasses a detection method of an AIM-related disease, which comprises a step of measuring the concentration of AIM in a sample collected from a subject.
In the present specification, "detection" means to examine a sample collected from a subject in order to obtain information necessary for the diagnosis, and the detection method of the present invention can be performed in, for example, a testing company and the like.
An explanation of the step of measuring the concentration of AIM in a sample collected from a subject is omitted, since it is the same as the aforementioned shinda method of the present invention.

### [Diagnostic kit for AIM-related disease]

A first embodiment of the diagnostic kit for AIM-related diseases of the present invention is a kit for performing the first embodiment of the aforementioned diagnostic method for AIM-related diseases of the present invention, and contains an anti-AIM antibody.
Said diagnostic kit contains a reagent and an apparatus necessary for measuring the concentration of AIM in a blood sample by an immunoassay utilizing an antigen antibody reaction between AIM and an anti-AIM antibody.

One embodiment of the above-mentioned diagnostic kit is for measuring the concentration of AIM by a sandwich ELISA method, and contains a microtiter plate; an anti-AIM antibody for trapping; an anti-AIM antibody labeled with alkaline phosphatase or peroxidase; and an alkaline phosphatase substrate (NPP etc.) or peroxidase substrate (DAB, TMB, microtiter plate OPD etc.).
Trapping antibody and labeled antibody recognize different epitopes.
Using such kit, first, a trapping antibody is immobilized on a microtiter plate, an appropriately treated and diluted blood sample is added and incubated, and the sample is removed and washed. Then, a labeled antibody is added and incubated, and the substrate is added to allow color development. Using a microtiter plate reader and the like, the color development is measured, and the concentration of AIM can be determined.

Another embodiment of the above-mentioned diagnostic kit is for measuring the concentration of AIM by a sandwich ELISA method using a secondary antibody, and contains a microtiter plate; an anti-AIM antibody for trapping; an anti-AIM antibody as a primary antibody; an alkaline phosphatase or peroxidase-labeled antibody to anti-AIM antibody as a secondary antibody; and an alkaline phosphatase (NPP etc.) or peroxidase substrate (DAB, TMB, OPD etc.).
Trapping antibody and primary antibody recognize different epitopes.
Using such kit, first, a trapping antibody is immobilized on a microtiter plate, an appropriately treated and diluted blood sample is added and incubated, and the sample is removed and washed. Then, the primary antibody is added and incubation and washing are performed, the enzyme-labeled secondary antibody is added and incubated, and the substrate is added to allow color development. Using a microtiter plate reader and the like, the color development is measured, and the concentration of AIM can be determined. Using a secondary antibody, the reaction is amplified and the detection sensitivity can be increased.

Each diagnostic kit further preferably contains necessary buffer, stop solution for enzyme reaction, microplate reader and the like.

The labeled antibody is not limited to an enzyme-labeled antibody, and may be an antibody labeled with a radioactive substance (²⁵I, ¹³¹I, ³⁵S, ³H etc.), a fluorescent substance (fluorescein isothiocyanate, rhodamine, dansyl chloride, phycoerythrin, tetramethyl rhodamine isothiocyanate, near infrared fluorescence material etc.), a luminescence substance (luciferase, luciferin, aequorin etc.), nanoparticles (gold colloid, quantum dot) and the like. It is also possible to use a biotinylated antibody as the labeled antibody, and add labeled avidin or streptavidin to the kit.

A still another embodiment of the diagnostic kit of the present invention is, for example, for the measurement of an AIM concentration by latex agglutination. This kit contains anti-AIM antibody sensitization latex, wherein a blood sample and an anti-AIM antibody are mixed, and aggregates are quantified by an optical method. The kit also preferably contains a coagulation reaction plate that visualizes a coagulation reaction.

The present invention also provides a novel use of an anti-AIM antibody, which is for diagnosis or detection of AIM-related diseases, or production of a diagnostic or test reagent for AIM-related diseases.
The present invention also provides a novel use of AIM or AIM fragment, which is for the production of an anti-AIM antibody for a diagnostic or test reagent for AIM-related diseases.

The present invention also encompasses an anti-AIM antibody useful for the diagnosis or detection of AIM-related diseases.
Specifically, it includes clones AIM-CL-6 (deposit number: NITE BP-1092) and AIM-CL-7 (deposit number: NITE BP-1093) deposited at Incorporated Administrative Agency National Institute of Technology and Evaluation Patent Microorganisms Depositary.

In addition, since AIM binds to CD36 in the body, changes of the expression level of CD36 are considered to also provide an effect equivalent to the change of the AIM level. Therefore, the present invention also provides a novel use of CD36, which is for the diagnosis or detection of AIM-related diseases. Examples

The present invention is specifically explained in the following based on the Examples, which are not to be construed as limitative.

### Example 1. Increase of blood AIM concentration due to obesity

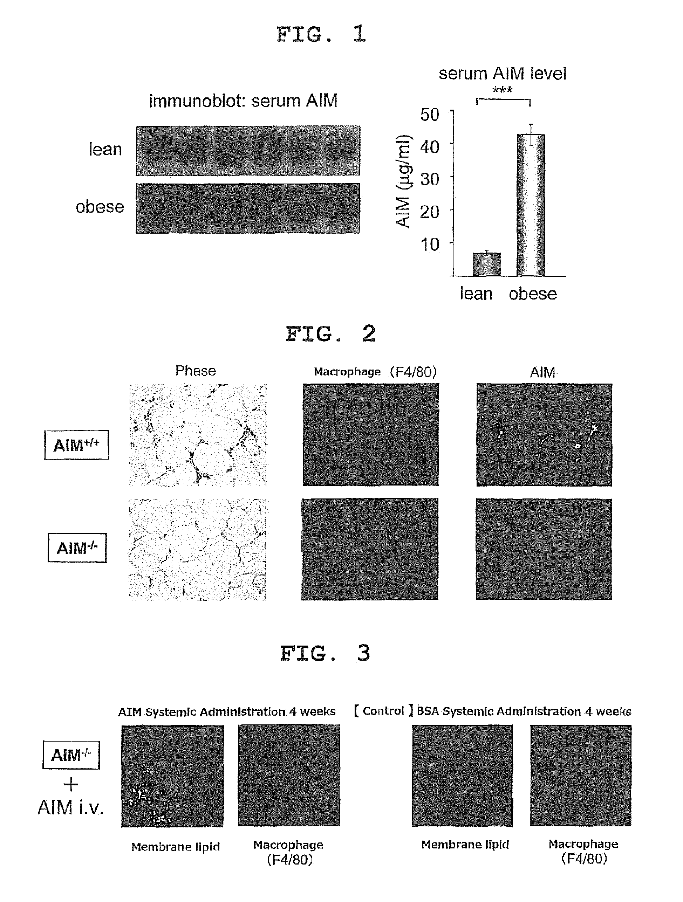

Serum AIM concentration of obese mice prepared by administering a high-fat diet (HFD, fat calorie: 60%) to C57BL. 6 (B6) mice for 20 weeks, and normal mice was measured. The results are shown in Fig. 1.
The serum AIM concentration of the obese mice (obese) was 4 times or more high as compared to that of the normal mice (lean).

### Example 2. Induction of infiltration of macrophage into adipose tissue by AIM

Visceral fat tissues were collected from AIM^{+/+} mice and AIM^{-/-} mice fed with HFD to become fat, and paraffin sections produced from the adipose tissues were stained with anti-macrophage monoclonal antibody (F4/80), anti-mouse AIM polyclonal antibody (SA-1) and anti-IL-6 antibody (MP520F3, R & D systems).
The results are shown in Fig. 2. As shown in Fig. 2, M1 macrophage infiltrated into the adipose tissue in the normal mice (AIM^{+/+}) expressing AIM, but infiltration of macrophage into adipose tissue was scarcely found in the AIM knockout mice (AIM^{-/-}).
As mentioned above, AIM has a suppressive function of macrophage apoptosis. Therefore, when macrophage is not detected in an adipose tissue of an AIM knockout mouse, there is a possibility that macrophage infiltrated into the adipose tissue and apoptosis occurred. To confirm this possibility, apoptosis of macrophage in the adipose tissues of obese AIM knockout mice and obese normal mice was measured. As a result, the level thereof was not different (data not shown).
Thus, it was confirmed that macrophage was not detected in the adipose tissue because macrophage did not infiltrate into the adipose tissue.

Then, rAIM was systemically administered to the AIM knockout mice by intravenous injection (50 µg/body/injection). After the administration was continued for 3 weeks, a section produced from the visceral fat tissue was stained with an anti-macrophage monoclonal antibody (F4/80).
The results are shown in Fig. 3. As shown in the Figure, it was observed that systemic administration of rAIM to an AIM knockout mice result in the infiltration of macrophages into adipose tissue.
From the results mentioned above, it is shown that AIM induces infiltration of macrophages into adipose tissue, and therefore, inhibition of AIM can suppress infiltration of macrophages into adipose tissue even in visceral fat-type obesity.

### Example 3. Elucidation mechanism of macrophage migration

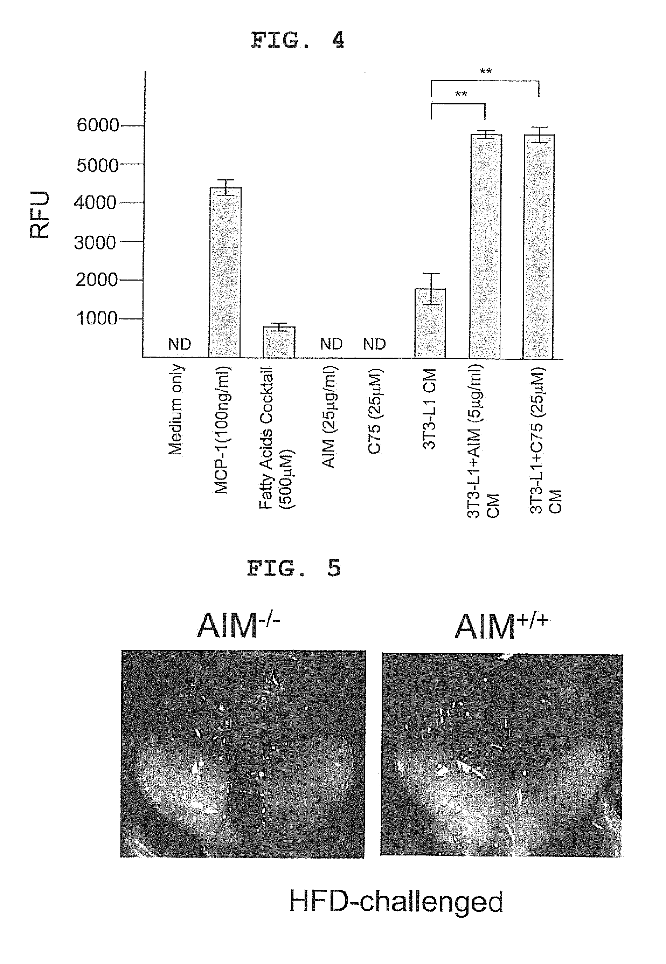

The migration capability of mouse macrophage cell line RAW264.8 (1 x 10⁵/well, 24 hr) was analyzed.
CytoSelect™ 96-Well Cell Migration Assay (CELL BIOLABS, Inc., 5 mm, Fluorometric format) was used. The results are shown in Fig. 4. Values obtained by subtracting the background (medium only) from respective RFU values are shown. Fatty Acids Cocktail consists of Myristoleic acid, Palmitic acid, Oleic acid and Linoleic acid.
Cell supernatant (3T3-L1 CM) obtained by culturing, for 6 days, mature 3T3-L1 adipocytes on day 8 from differentiation induction, supernatant obtained by co-culture with rAIM (3T3-L1+AIM CM), and supernatant obtained by co-culture with C75 (3T3-L1+C75 CM) were used. CM: conditioned medium, ND: not detected. **: p<0.01
As shown in the below-mentioned Reference Examples, AIM induces lipid droplet fusion in mature adipocytes. As a result, free fatty acid and glycerol derived from lipid droplet are released in the culture supernatant.
Combined with the results of the macrophage migration experiment, it is strongly suggested that AIM having an increased blood concentration along with the obesity induces lipid droplet fusion in adipose tissue, which induces infiltration of macrophages into adipose tissue.

### Example 4. Suppression of decrease in glucose metabolism of obese AIM knockout mice

Wild-type mice and AIM knockout mice were loaded with HFD for 12 weeks, and glucose metabolism was measured and an insulin sensitivity test was performed, before (Lean) and after (Obese) the loading, by a glucose tolerance test and an insulin tolerance test.
In the glucose tolerance test, the blood glucose level was measured every 30 min for 120 min after the glucose loading (3 g/kg body; intraperitoneal injection), and in the insulin tolerance test, the blood glucose level was measured every 30 min for 120 min after the insulin loading (0.75 U/kg body; intraperitoneal injection).
The insulin sensitivity test was performed by administering insulin to AIM^{+/+} mice and AIM^{-/-} mice after loading with HFD (0.75 U/kg body; intravenous injection), collecting tissues (white adipose tissue, gastrocnemius muscle and liver) 2 hr later, purifying protein, and measuring phosphorylated AKT and phosphorylated GSK3β by Western blotting.
In addition, the body weight and total amount of fat of each mouse were measured after HFD loading.

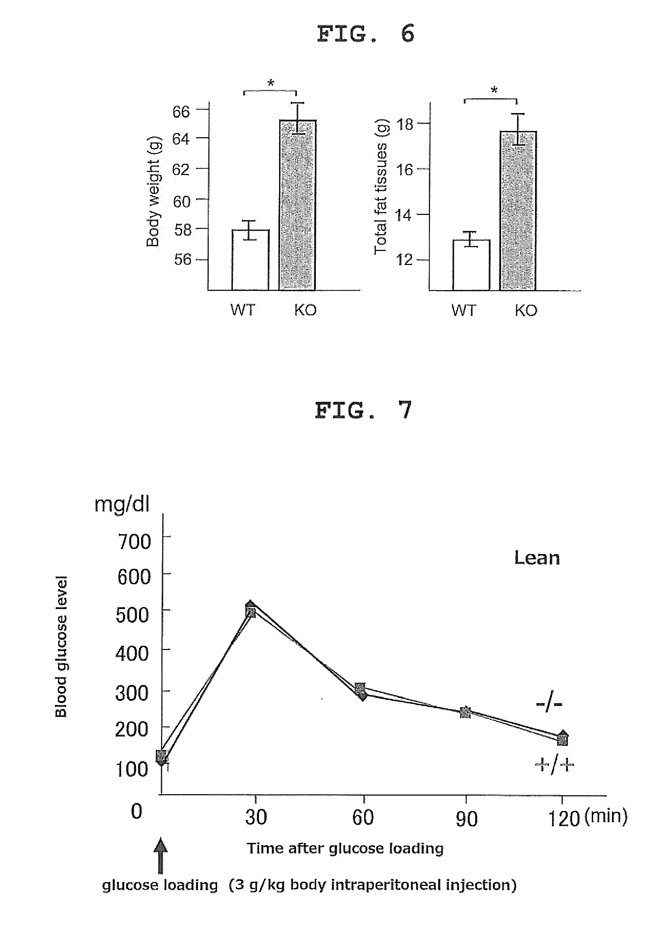

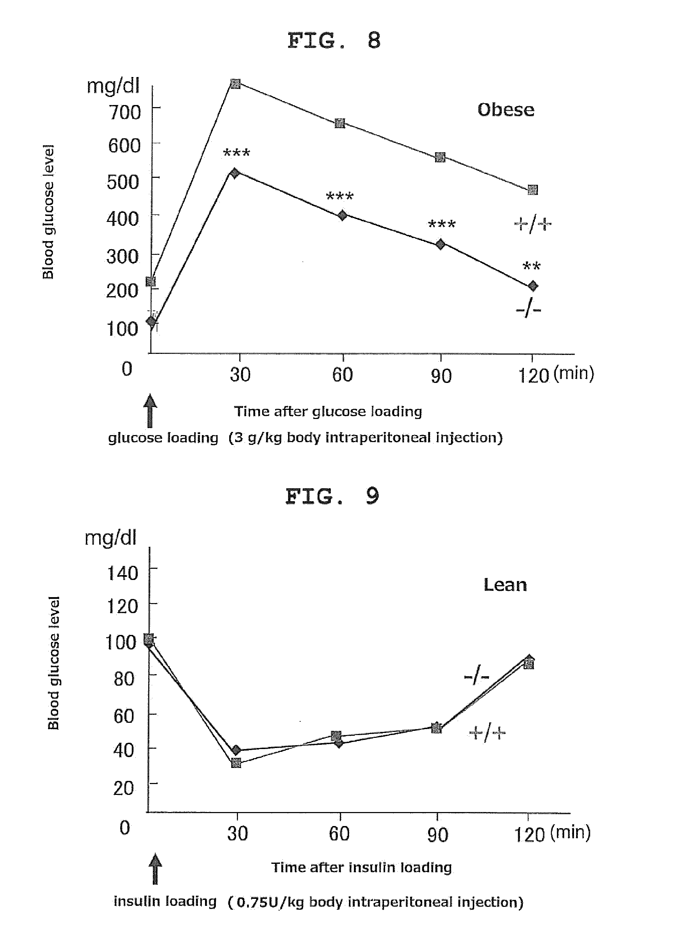

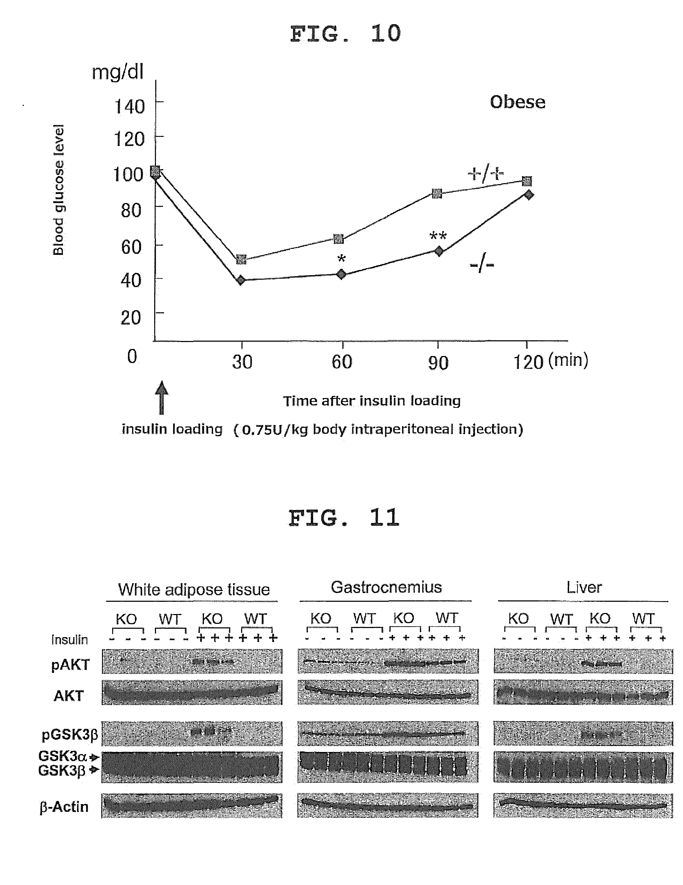

Fig. 5 shows the results of anatomy after loading, Fig. 6 shows the measurement results of body weight and total amount of fat, Figs. 7 - 10 show the measurement results of glucose metabolism, and Fig. 11 shows the results of the insulin sensitivity test.
As shown in Figs. 5 and 6, HFD loading caused visceral-type obesity in both of the AIM knockout mice (AIM^{-/-}) and normal mice (AIM^{+/+}). Increase in the body weight and adipose tissue amount was more remarkable in the AIM knockout mice.
However, as shown in Figs. 7 - 10, glucose metabolism markedly decreased in the obese normal mice, but the glucose metabolism did not decrease in the obese AIM knockout mice.
Furthermore, as shown in Fig. 11, phosphorylated AKT and phosphorylated GSK3β were expressed in the AIM^{-/-} mice after insulin administration, and it was confirmed that signal transduction via an insulin receptor functioned, that is, the insulin sensitivity was normal. On the other hand, phosphorylated AKT and phosphorylated GSK3β were not detected in the wild-type mice, thus confirming the presence of insulin resistance.
In addition, it was also confirmed by a hyperinsulinemic-euglycemic clamp test that the AIM^{-/-} mice had normal insulin sensitivity (data not shown).
The above-mentioned results support that, in obese AIM knockout mice, macrophage does not infiltrate into adipose tissue, and therefore, inflammation reaction of adipose tissue and the whole body is suppressed and the insulin sensitivity does not decrease.
The isles of Langerhans in the pancreas were separated from the obese AIM knockout mice or normal mice, and production and secretion of insulin was measured by glucose loading. As a result, no difference was found between the both. Therefore, it was confirmed that the difference in the glucose tolerance is not based on insulin production or secretion but on difference in insulin sensitivity.

### Reference Example 1. Expression of AIM in macrophage in adipose tissue

Visceral fat tissues were collected from lean mice (lean) obtained by feeding a general feed to wild-type B6 mice and obese mice (obese) obtained by feeding a high-fat diet (HFD, fat calorie 60%) for 20 weeks, and paraffin sections produced from the adipose tissues were double stained with anti-macrophage monoclonal antibody (F4/80), anti-mouse AIM polyclonal antibody (SA-1) and anti-IL-6 antibody (MP520F3, R & D systems).

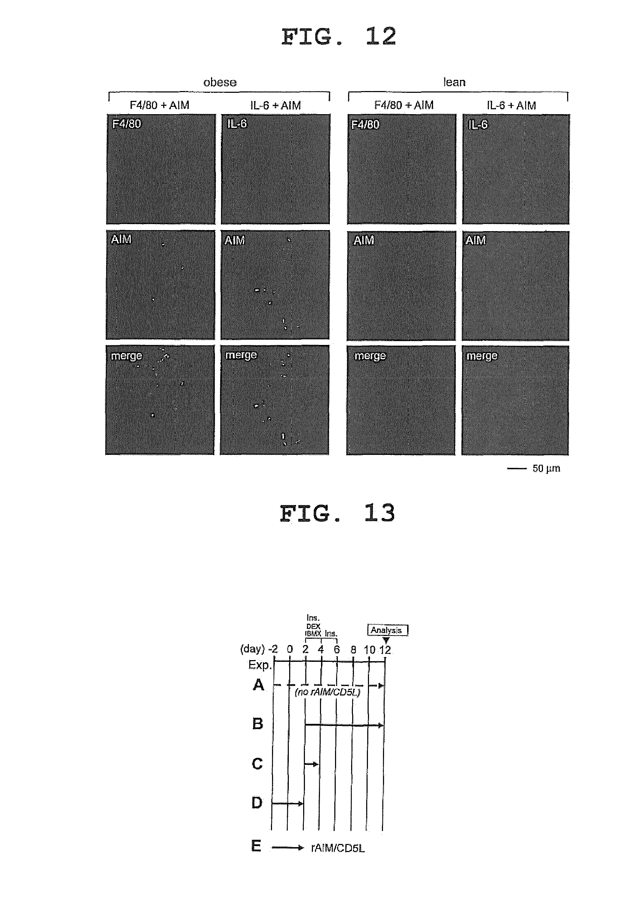

Fig. 12 shows one example of the results of observation of the stained section with a fluorescence microscope.
The macrophages in adipose tissue of the obese mice, which was stained with an anti-macrophage antibody, were also stained with an AIM specific antibody (obese left lane). Since AIM positive macrophages were also positive for IL-6 (obese right lane), they were considered as inflammatory macrophages (M1).
On the other hand, the macrophages in the adipose tissue collected from the lean mice were negative for both AIM and IL-6.
To confirm that AIM was not expressed in adipocyte, adipose tissue derived from obese mice (epididymal adipose tissue was used as representative visceral tissue) was fractionated after collagenase treatment, and expression of AIM was examined by RT-PCR. However, AIM expression was not found in purified adipocyte. Furthermore, 3T3-L1 cells were treated with insulin, DEX and IBMX, and AIM expression was examined. As a result, expression was not found.
From the above-mentioned results, it was confirmed that macrophage that infiltrated into adipose tissue strongly expressed AIM.

### Reference Example 2. Suppressive function on differentiation of preadipocyte into adipocyte by AIM

### [Evaluation by lipid droplet formation]

To examine the function of AIM produced by macrophages that infiltrated into adipose tissue on the surrounding adipocytes, an experiment was performed to load AIM in the culture process for differentiation of 3T3-L1 preadipocytes into mature adipocytes.
As shown in Fig. 13, 3T3-L1 cells were cultured in four schedules A - D. In (A), AIM was not loaded, in (B), AIM was loaded for 10 days from the start of the differentiation induction stimulation (day 2-day 12), in (C), AIM was loaded only in the initial stage of differentiation induction stimulation (day 2-day 4), and in (D), AIM was loaded only in the clonal expansion period before differentiation induction (day(-2)-day 2).

As AIM, recombinant AIM protein (rAIM) of mouse was used. rAIM was prepared by culturing human-derived HEK293T cells transfected with a vector expressing mouse AIM (pCAGGS-mAIM-HA plasmid) in a serum-free medium (FreeStyle™ 293 Expression Medium; Invitrogen). rAIM was purified from the culture supernatant thereof using an anti-HA antibody column. In other Reference Examples shown below, AIM used was a similar rAIM protein. AIM was loaded by adding AIM to a culture medium at a concentration of 5 µg/ml.

In addition, differentiation of 3T3-L1 cells was induced as shown in Fig. 13 by first proliferating 3T3-L1 cells by cultivating for 4 days (day(-2)-day 2), after which cultivating in a culture medium containing insulin, dexamethasone (DEX) and isobutylmethylxanthine (IBMX) for 2 days (day 2-day 4) to start differentiation induction stimulation, and further cultivating in a culture medium containing insulin for 2 days (day 4-day 6).

After the differentiation induction stimulation period (day 2 - day 6), the cells were continuously cultured in a culture medium free of such differentiation induction stimulation factors. Then, the cells on day 10 day from the start of the differentiation induction stimulation (day 12) were stained with oil-red-O, and the state of differentiation of 3T3-L1 cells into mature adipocytes was observed.

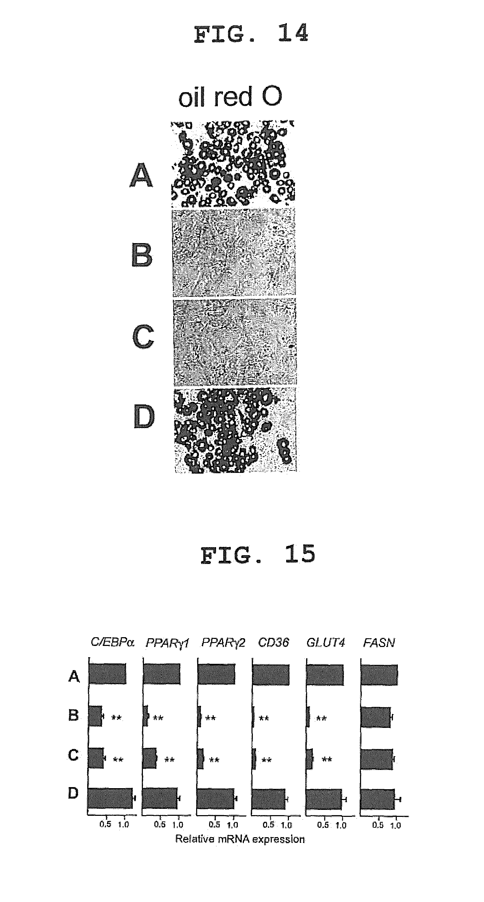

Fig. 14 shows microscopic photographs of the respective stained cells of 4 schedules A - D.

As shown in Fig. 14, when AIM was loaded only in the initial stage (day 2 - day 4) of differentiation induction stimulation in schedule C, cells having lipid droplet were scarcely observed, and differentiation of 3T3-L1 cells was nearly completely suppressed. In other words, AIM suppressed differentiation of 3T3-L1 cells in the presence of the aforementioned differentiation inducer.

Even when AIM was removed from a culture medium containing a differentiation inducer and AIM, by using a purification column after preparation of the medium, the ability to differentiate the 3T3-L1 cells by said differentiation inducer was not lost (data not shown). Therefore, no substantive chemical interaction was considered to be present between the differentiation inducer and AIM.

In addition, when AIM was loaded for 10 days (day 2-day 12) from the start of the differentiation induction stimulation in schedule B, cells having lipid droplet were scarcely observed, and the differentiation of 3T3-L1 cells was nearly completely suppressed. Furthermore, such differentiation suppressive effect of AIM was confirmed to depend on the concentration of AIM (data not shown). When the concentration of AIM loaded on the 3T3-L1 cells was decreased to 1 µg/ml and 0.1 µg/ml, the differentiation suppressive effect of AIM also decreased.

Moreover, the number of dead cells did not increase due to AIM loading. Therefore, it was considered that suppression of differentiation by AIM did not induce cell death but was caused by decrease in the number of 3T3-L1 cells that differentiated into mature adipocytes.

On the other hand, when AIM was loaded only before differentiation induction (day(-2) - day 2) in schedule D, it was confirmed that almost all cells had lipid droplet, and almost all 3T3-L1 cells differentiated into mature adipocytes, as in the case of no loading of AIM in schedule A. That is, even when AIM was loaded only before differentiation induction, differentiation of 3T3-L1 cells into mature adipocytes was not suppressed. However, when AIM was continuously loaded from before differentiation induction through differentiation induction period and thereafter (day (-2) - day 12), differentiation of 3T3-L1 cells into adipocytes was completely suppressed (data not shown).

When human rAIM was used for AIM loading, differentiation of mouse-derived 3T3-L1 cells was similarly suppressed. This shows that the function of AIM is interchangeable between human and mouse.

### [Evaluation by expression of adipocyte markers]

The cells cultured in schedules A - D shown in Fig. 13 were recovered on day 10 from the start of the differentiation induction stimulation (day 12), and expression of adipocyte markers (C/EBPα, PPARγ1, PPARγ2, CD36, Glut4) at mRNA level was measured using 7500 Fast Real-Time PCR system (Invitrogen; CA, USA) by ΔΔC_{T} method. The primers used are shown in Table 1.

**Table 1**

| | |
|---|---|
| f-PPARγ1 | CAAGAATACCAAAGTGCGATCAA (SEQ ID NO:2) |
| r-PPARγ1 | GAGCTGGGTCTTTTCAGAATAATAAG (SEQ ID NO:3) |
| f-PPARγ2 | CCAGAGCATGGTGCCTTCGCT (SEQ ID NO:4) |
| r-PPARγ2 | CAGCAACCATTGGGTCAGCTC (SEQ ID NO:5) |
| f-C/EBPα | AGCAACGAGTACCGGGTACG (SEQ ID NO:6) |
| r-C/EBPα | TGTTTGGCTTTATCTCGGCTC (SEQ ID NO:7) |
| f-CD36 | TTGTACCTATACTGTGGCTAAATGAGA (SEQ ID NO:8) |
| r-CD36 | CTTGTGTTTTGAACATTTCTGCTT (SEQ ID NO: 9) |
| f-Glut4 | GACGGACACTCCATCTGTTG (SEQ ID NO:10) |
| r-Glut4 | GCCACGATGGAGACATAGC (SEQ ID NO:11) |

The results are shown in Fig. 15. It was shown that adipocyte markers were expressed and preadipocytes were differentiated into adipocytes in schedules A and D. On the other hand, expression of adipocyte markers were suppressed in schedules B and D in which AIM was administered simultaneously with differentiation induction stimulation, and suppression of differentiation into adipocyte was suggested. The results are consistent with schedules A and D where lipid droplet was formed and schedules B and D where it was not formed.

As mentioned above, AIM suppresses differentiation of preadipocytes into adipocytes. In addition, the level of differentiation can be examined easily by observation of lipid droplet formation or detection of adipocyte marker, preadipocyte marker or mesenchymal stem cell marker.
Therefore, as a result of lipid droplet formation or detection of marker, when the differentiation induction efficiency increases when AIM and a candidate compound are added together as compared to the addition of AIM alone, said candidate compound can be evaluated to be a compound that suppresses the function of AIM, and when the differentiation induction efficiency decreases, said candidate compound can be evaluated to promote the function of AIM.

### Reference Example 3. Function of lipid droplet fusion of adipocyte by AIM

### [Evaluation by oil-red-O]

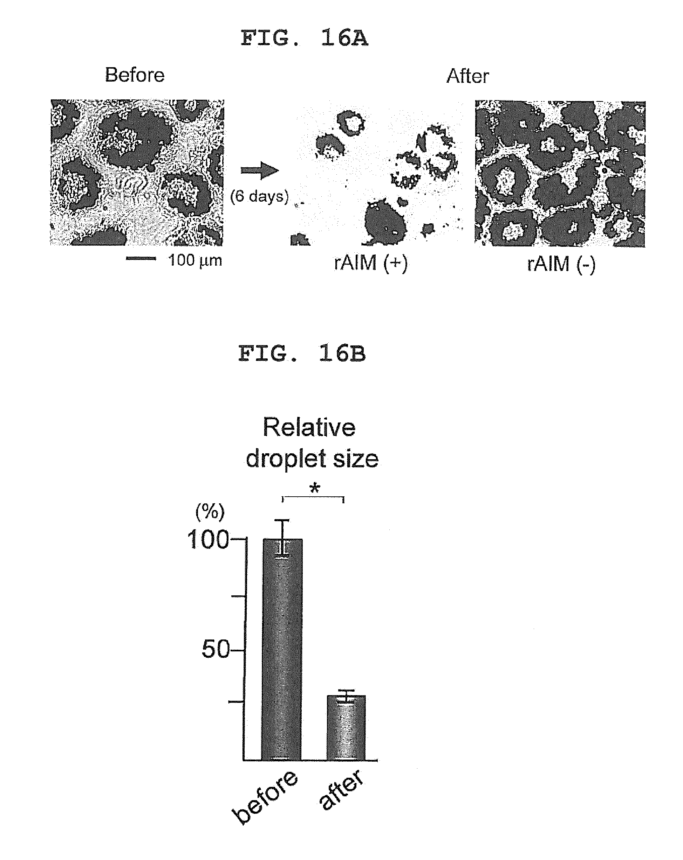

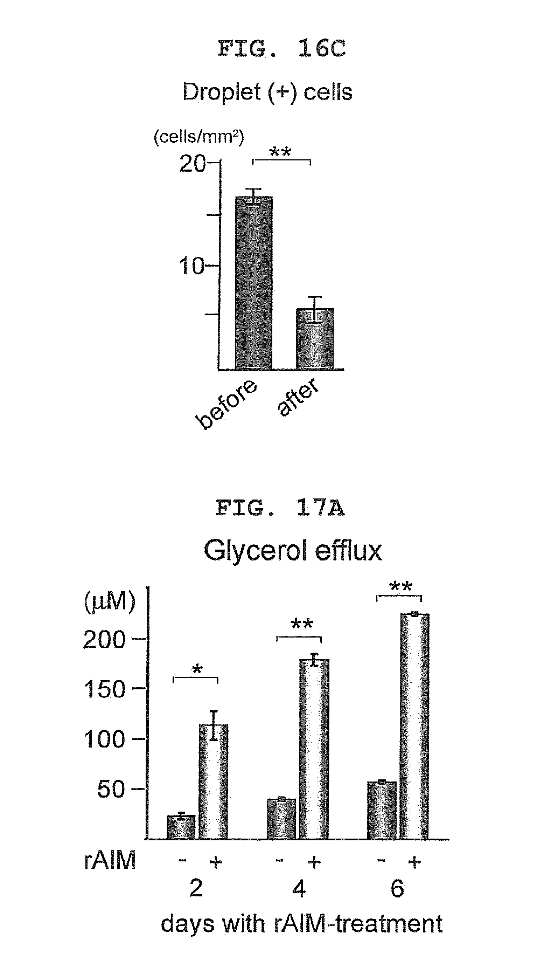

The differentiated 3T3-L1 cells were loaded with rAIM (5 µg/ml) according to the schedule shown in Fig. 13, E. The oil-red-O staining was performed before and after rAIM loading. Representative photograph is shown in Fig. 16A. The lipid droplet in the cell markedly decreased after rAIM loading followed by 6 days of culture (rAIM(+)).
The relative size of lipid droplet (Relative droplet size) was determined from an average diameter of 50 lipid droplets. The error bar shows standard error. The number of cells containing lipid droplets per unit area was measured in 5 different fields and the average was determined. Each result is shown in Figs. 16B, C.
The relative size of lipid droplets and the number of lipid droplet-containing cells significantly decreased.
The results suggest that rAIM induces lipid droplet fusion, and glycerol and free fatty acids contained in the lipid droplets were released in the supernatant.

### [Evaluation by amount of glycerol or free fatty acids in culture supernatant]

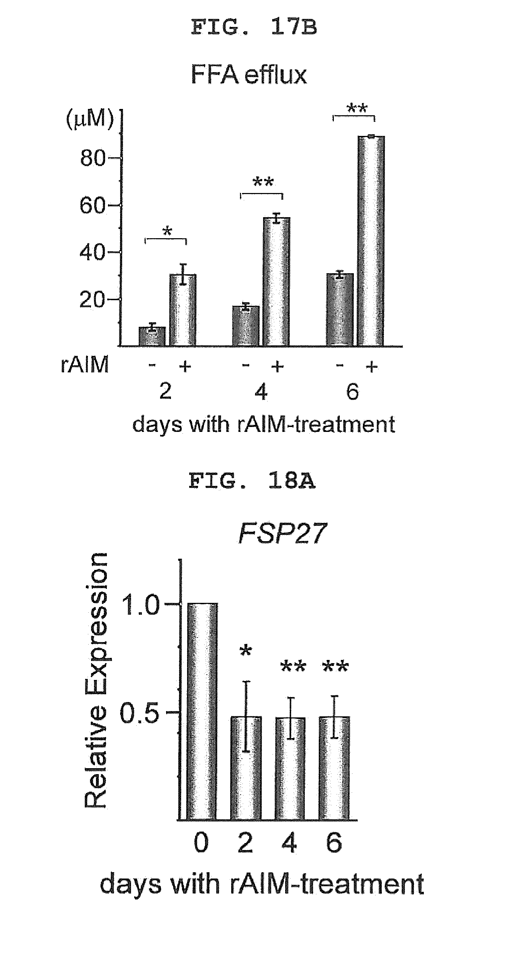

rAIM was loaded (5 µg/ml) on 3T3-L1 adipocytes, the amount of glycerol or free fatty acid released in the culture supernatant was measured on day 2, day 4, and day 6. After rAIM loading, the cells were washed twice with PBS, and incubated in a serum-free medium (FreeStyle™ 293 Expression Medium; Invitrogen) for 5 hr. The buffer was recovered, and the measurement was performed using a glycerol assay kit and a fatty acid assay kit (both Bio Vision Inc.) and according to the manual.
The results are shown in Fig. 17. When rAIM was added before cultivation, the amounts of glycerol and free fatty acids in the culture supernatant significantly increased.
The results suggest that rAIM induced lipid droplet fusion, and glycerol and free fatty acids contained in the lipid droplet were released in the supernatant.

### [Evaluation by expression of lipid droplet formation-related genes]

rAIM was loaded (5 µg/ml) on 3T3-L1 adipocytes, mRNA of lipid droplet formation-related genes (FSP27, Perilipin, and Adipophilin) was measured on day 2, day 4, and day 6 using 7500 Fast Real-Time PCR system (Invitrogen; CA, USA) and by the ΔΔC_{T} method (each n=3).
Similarly, mRNA of PREF-1 of preadipocyte marker was also measured. The measurement value was standardized with GAPDH. The primers used are shown in Table 2.

**Table 2**

| | |
|---|---|
| f-Fsp27 | CTGGAGGAAGATGGCACAATCGTG (SEQ ID NO:12) |
| r-Fsp27 | CAGCCAATAAAGTCCTGAGGGTTCA (SEQ ID NO: 13) |
| f-Perilipin, | TGCTGGATGGAGACCTC (SEQ ID NO:14) |
| r-Perilipin | ACCGGCTCCATGCTCCA (SEQ ID NO:15) |
| f-Adipophilin | AAGCATCGGCTACGACGACAC (SEQ ID No:16) |
| r-Adipophilin | GGACAGTCTGGCATGTAGTCTGGA (SEQ ID NO:17) |
| f-GAPDH | AACTTTGGCATTGTGGAAGG (SEQ m NO:18) |
| r-GAPDH | GGATGCAGGGATGATGTTCT (SEQ ID NO:19) |
| f-PREF1 | CGGGAAATTCTGCGAAATAG (SEQ ID NO:20) |
| r-PREF1 | TGTGCAGGAGCATTCGTACT (SEQ ID No:21) |

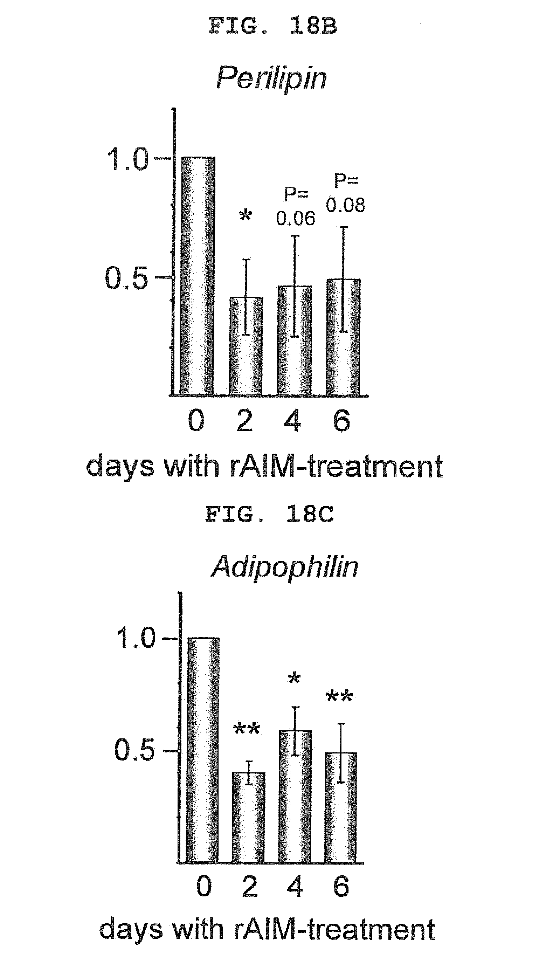

The results are shown in Fig. 18. Relative expression level was determined with that on day 0 as 1.0. The error bar shows standard error.
The expression of lipid droplet formation-related genes had already decreased markedly on day 2 after rAIM treatment. On the other hand, the expression of preadipocyte markers did not increase during rAIM treatment. This suggests that AIM does not cause dedifferentiation of mature adipocyte.

As mentioned above, AIM fuses lipid droplet in mature adipocyte. The level of lipid droplet fusion can be examined easily by observation with a microscope, measurement of glycerol or free fatty acid level of the culture supernatant, measurement of the expression of lipid droplet formation-related gene and the like.
Therefore, as a result of detection of glycerol and the like in the culture supernatant, when the lipid droplet fusion efficiency decreases when AIM and a candidate compound are added together as compared to the addition of AIM alone, said candidate compound can be evaluated to be a compound that suppresses the function of AIM, and when the lipid droplet fusion efficiency increases, said candidate compound can be evaluated to promote the function of AIM.

### Reference Examples 4. Function on reducing the size of adipocytes by AIM

### [Evaluation by HE staining]

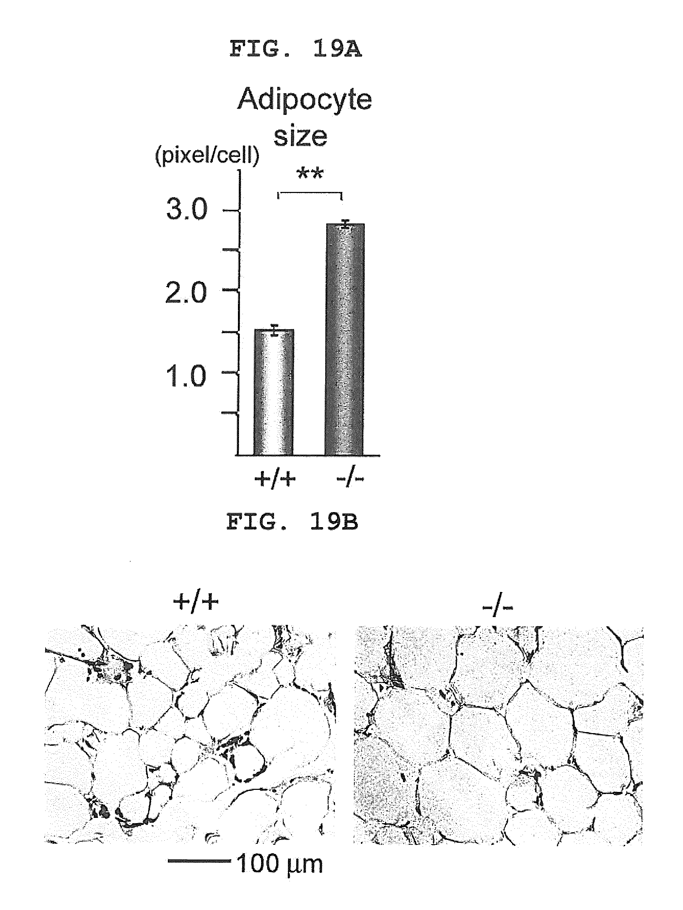

AIM^{+/+} mice (+/+) and AIM^{-/-} mice (-/-) (see non-patent document 1) were fed with a high-fat diet (HFD) for 20 days, and a section of adipose tissue was stained with HE. In various fields of a microscope, the distance among 50 adipocytes was measured, and mean±standard error was determined. The results are shown in Fig. 19A, and representative photograph is shown in Fig. 19B. The adipocytes of AIM^{-/-} mice were significantly large as compared to AIM^{+/+} mice.

As mentioned above, AIM reduces the size of adipocytes. The level of reduction can be examined easily by a microscope.
Therefore, as a result of measurement of the size, when the cell size increases when AIM and a candidate compound are added together as compared to the addition of AIM alone, said candidate compound can be evaluated to be a compound that suppresses the function of AIM, and when the cell size decreases, said candidate compound can be evaluated to promote the function of AIM.

### Reference Example 5. AIM uptake into adipocyte via CD36 on cell surface

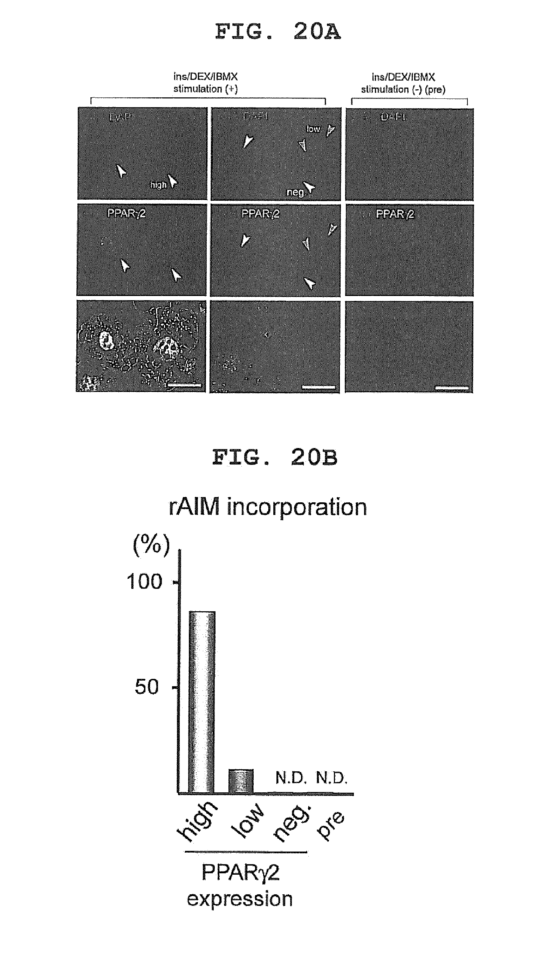

Differentiated 3T3-L1 cells (ins/DEX/IBMX stimulation (+)) and undifferentiated 3T3-L1 cells (ins/DEX/IBMX stimulation (-)) were incubated with rAIM (5 µg/ml) for 3 hr, and stained with AIM, PPARγ2, and DAPI. For staining with PPARγ2, a rabbit anti-PPARγ2 polyclonal antibody (Abcam plc) was used. The results are shown in Fig. 20A.
The upper panel shows the results of nucleus stained with AIM and DAPI, the middle panel shows the results of staining with AIM and PPARγ2, and the lower panel shows the results of phase contrast image of staining with AIM, PPARγ2 and DAPI. It was observed that AIM was dispersed in the cytoplasm of adipocyte.
As is clear from the lower panel, the cells highly expressing PPARγ2 contains many lipid droplets. The cells highly expressing PPARγ2 are sufficiently differentiated mature adipocytes. The right lane indicated as "pre" shows undifferentiated 3T3-L1 preadipocyte without differentiation induction stimulation.
Fig. 20B shows the number of cells containing rAIM per 100 cells which was obtained by classifying the cells according to the expression level of PPARγ2. As shown in the Figure, it was found that mature adipocytes highly expressing PPARγ2 showed efficient rAIM uptake, and the cells showing low expression of PPARγ2, which were not sufficiently differentiated, showed markedly low rAIM uptake efficiency. The preadipocytes (pre) free of differentiation induction stimulation showed no rAIM uptake.

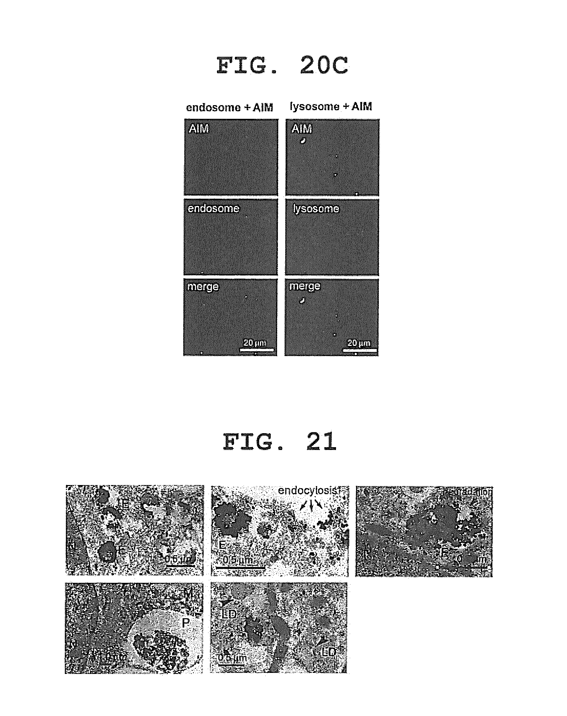

In addition, 3T3-L1 adipocytes were treated with rAIM for 3 hr, stained with AIM and endosome (FM 1-43FX (Invitrogen Corporation) was used), AIM and lysosome (Lyso Tracker Red DND-99 (Invitrogen Corporation) was used), and observed under a confocal microscope. The results are shown in Fig. 20C.
rAIM incorporated into adipocyte was co-localized with endosome, and co-localization with lysosome was not observed.

Using the same sample, AIM was labeled with gold nanoparticles, and observed under an electron microscopy. Specifically, the cells pre-treated with normal goat serum for 30 min were fixed with para-formaldehyde, and incubated overnight with SA-1 rabbit anti-AIM polyclonal antibody (1:600 dilution). Then, they were reacted with goat anti-rabbit IgG covalently bound with 1 nm gold nanoparticles (1:200; Nanoprobes, Inc.). The cells were silver enhanced using HQ silver (Nanoprobes, Inc.), osmificated, dehydrated, and directly embedded in Epon (Nisshin EM Corporation.). An extremely thin section was prepared, stained with uranyl acetate and lead citrate, and observed with an electron microscope (H-7100; Hitachi Inc.).
The results are shown in Fig. 21. E shows endosome, P shows phagosome or phagolysosome, M shows mitochondria, N shows nucleus, and LD shows lipid droplet.
rAIM labeled with gold nanoparticles has an endosome-like structure, and particularly accumulated near the limiting membrane thereof (upper left panel). In the cellular membrane, endocytosis of rAIM was observed (upper center panel). Some particles containing rAIM were found near the nucleus, and considered to be late endosome (upper right panel). The late endosome is denatured, along with which rAIM is released in the cytoplasm.
AIM was not detected in phagosome and phagolysosome having a large diameter and an irregular shape, mitochondria and lipid droplet (lower panel).
Putting the above results together, it is suggested that AIM is incorporated into adipocyte by endocytosis, and exhibits function in the cell.

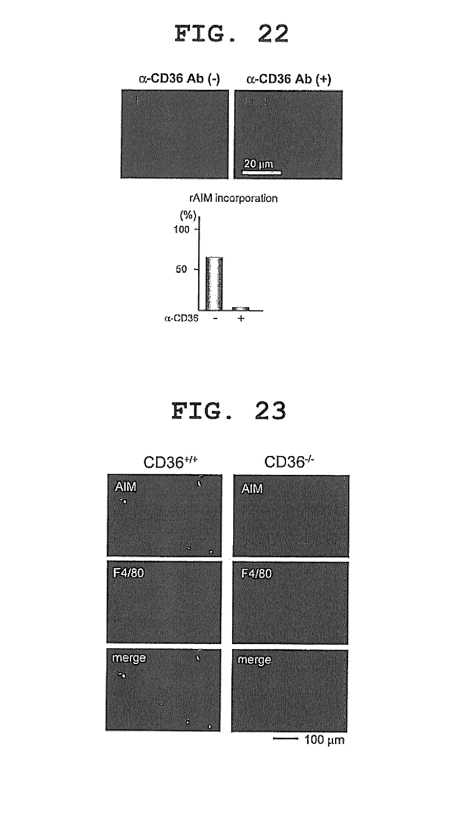

Then, the cell surface molecule involved in AIM uptake was specified. Attention was paid to CD36 since it promotes uptake of lipoprotein, fatty acid and the like and is expressed in both adipocytes and macrophages, which are the target of AIM function.
3T3-L1 adipocytes were treated with a CD36 neutralizing antibody (Abcam plc; Clone JC63.1 mouse IgA) to inhibit the endocytosis of rAIM. Fig. 22 shows representative photographs of treatment and no treatment with neutralizing antibody, and the number of cells that incorporated rAIM per 100 cells.
As shown in the Figure, the treatment with a CD36 neutralizing antibody markedly suppressed rAIM uptake.

Then, CD36^{+/+} mice (wild-type) and CD36^{-/-} mice (Febbraio et al., J. Biol. Chem., 1999, 274:19055-19062) were intravenously injected with rAIM (300 µg/mouse in PBS), sacrificed 16 hr later, and a section was prepared from adipose tissue. AIM and macrophage F4/80 in the section were stained. The results are shown in Fig. 23.
As shown in the Figure, it was shown that AIM signal markedly decreased in CD36^{-/-} adipocyte, and MAIM uptake capacity was lost in CD36^{-/-} adipocyte.
These results strongly suggest that CD36 causes rAIM uptake.

As mentioned above, AIM is incorporated into adipocytes via CD36 on the cell surface. The level of AIM uptake by adipocytes can be examined easily by, for example, visualizing intracellular AIM using a detectably labeled anti-AIM antibody.
Therefore, using this method, the action mechanism of the candidate compound that suppresses the function of AIM can be clarified, and the effect of said compound can be verified.
For example, when the intracellular uptake of AIM decreases when AIM and a candidate compound that suppresses the function of AIM are added together as compared to the addition of AIM alone, said candidate compound is considered to suppress the function of AIM by inhibiting the intracellular uptake of AIM.

### Reference Example 6. Blinding of AIM and FAS

### [Evaluation by immunoprecipitation]

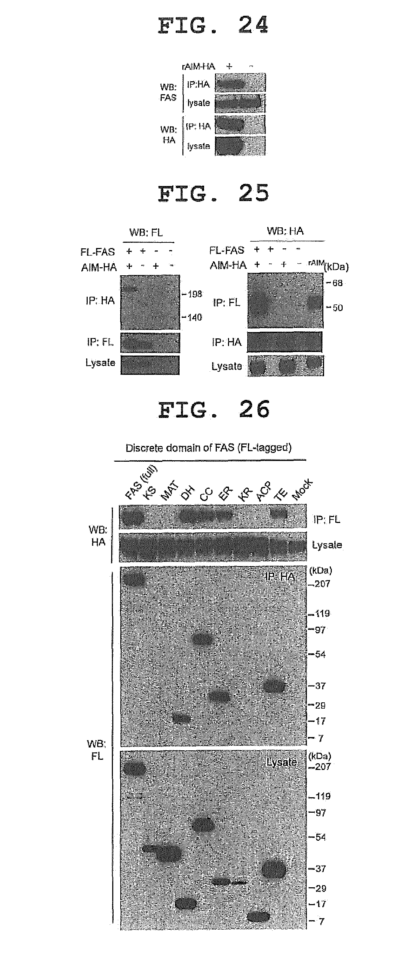

rAIM labeled with HA-tag was directly injected to adipose tissue of AIM^{-/-} mice (at several points, 100 µg in total). After 3 hr, to confirm binding of rAIM-HA and endogenous FAS, co-immunoprecipitation by anti-HA antibody was performed using adipose tissue. The presence of FAS in the precipitate was analyzed by Western blotting (WB).
The results are shown in Fig. 24. As shown in the Figure, it was confirmed that FAS and rAIM were coprecipitated, and incorporated AIM and FAS in the cytoplasm were bound.

In HEK 293T cells, rAIM labeled with HA-tag and FAS labeled with FLAG-tag were co-expressed, and the binding of AIM and FAS was confirmed by co-immunoprecipitation using an anti-Flag antibody or anti-HA antibody.
The results are shown in Fig. 25. rAIM and FAS coprecipitated, and the binding capacity of AIM to FAS was shown.

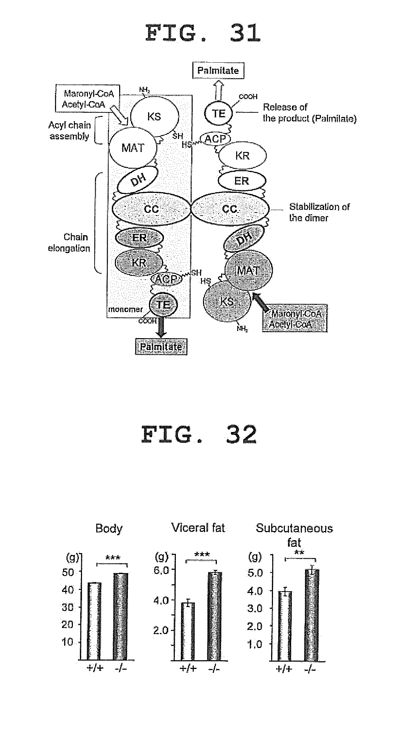

Then, the AIM binding region in FAS was examined. FAS contains 8 domains of keto acyl synthase (KS), malonyl/acetyl transferase (MAT), dehydrase (DH), central core (CC), enoylreductase (ER), keto reductase (KR), acyl carrier protein (ACP) and thioesterase (TE).
The structure of FAS is shown in Fig. 31.
The N-terminal of each domain of FAS was labeled with Flag sequence, and expressed in HEK293T cells where AIM-HA is stably expressed. The binding of AIM-HA and each domain of FAS was confirmed by co-immunoprecipitation using an anti-Flag antibody or anti-HA antibody.
The results are shown in Fig. 26. It was found that the domains bound to AIM were ER, DH, TE and CC.
The sequence encoding full-length FAS cDNA (nucleotide: +1 - 7515) labeled with FLAG-tag was constructed using a part of the cDNA clone provided by Dr. Ohara (Kazusa DNA Research Institute) and some fragments cloned by RT-PCR and pFLAG-CMV2 vector (Sigma).
A cDNA fragment encoding KS, MAT, DH, CC, ER, KR, ACP or TE was prepared by subcloning to pFLAG-CMV2 vector by using full-length FAS cDNA as a template.

### [Suppression of FAS enzyme activity by AIM]

The influence of AIM on FAS activity of adipocyte or adipose tissue was examined. FAS activity was measured by the method of Kelley (Kelley et al., Biochem. J., 1986, 235:87-90) with slight modification. 3T3-L1 adipocytes, or lysates of adipose tissue of obese mice was mixed with acetyl-CoA and NADPH (0.2 M calcium phosphate buffer [pH 7.0] containing 0.4 mM EDTA), and the mixture was placed in a spectrophotometer cuvette and maintained at 30°C. 20 µl of malonyl-CoA solution (0.2 mM) was added to start an enzyme reaction and decrease of OD was measured at a chart speed 2 cm/min. Based on the results, the enzyme activity of FAS was evaluated using the molar extinction coefficient of oxidized NADPH as 6220.

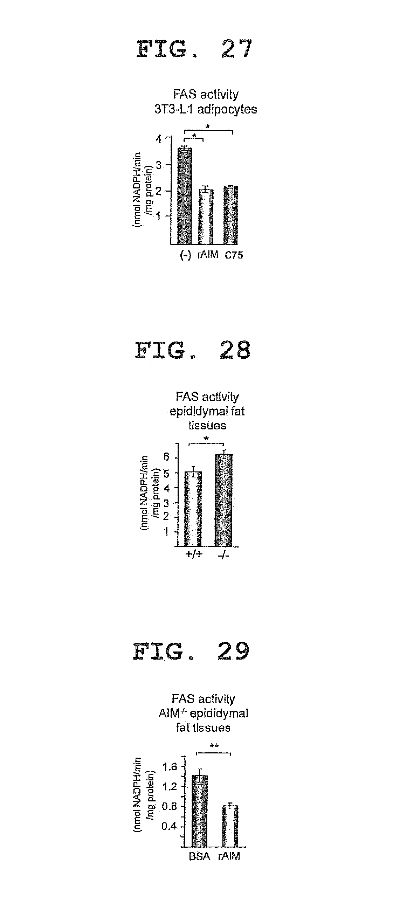

First, the FAS activity of 3T3-L1 cells treated for 6 days in the presence or absence of rAIM (5 µg/ml), and in the presence of C75 (25 µM) was measured. The results are shown in Fig. 27. Moreover, the FAS activity of adipose tissues of AIM^{+/+} mice and AIM^{-/-} mice and the FAS activity of adipose tissue of AIM^{-/-} mice administered with rAIM or BSA 3 hr before by intraadipose topical injection are shown in Figs. 28 and 29, respectively.
All mice were fed with HFD for 20 weeks. The samples were dissolved (lysed) and the FAS activity was measured. The data was converted to that based on FAS protein, according to the results of WB performed using the same sample. In each group, n was 6, and the error bar shows standard error.
As shown in Fig. 27, FAS activity in 3T3-L1 adipocytes was markedly suppressed by the rAIM treatment. The level of suppression was the same as C75 that specifically inhibits FAS.
As compared to AIM^{+/+} mice, adipose tissue significantly increased in vivo in AIM^{-/-} mice (Fig. 28). In addition, direct administration of rAIM of adipose tissue of AIM^{-/-} mice decreased FAS activity.

As mentioned above, AIM binds to FAS and suppresses its activity.
The level of binding of AIM and FAS can be examined easily by immunoprecipitation and the like. Therefore, using this method, the action mechanism of the candidate compound that suppresses the function of AIM can be clarified, and the effect of said compound can be verified. For example, when the binding of AIM and FAS decreases when AIM and a candidate compound that suppresses the function of AIM are added together as compared to the addition of AIM alone, said candidate compound is considered to suppress the function of AIM by inhibiting the binding of AIM and FAS.
The level of suppression of activity can be examined easily by a general method for measuring FAS activity. Therefore, using this method, the action mechanism of the candidate compound that suppresses the function of AIM can be clarified, and the effect of said compound can be verified. For example, when the suppression of FAS activity is suppressed when AIM and a candidate compound that suppresses the function of AIM are added together as compared to the addition of AIM alone, said candidate compound is considered to suppress the function of AIM by inhibiting the FAS activity suppressive function of AIM.

### Reference Example 7. changes of body weight and fat amount by AIM in vivo)

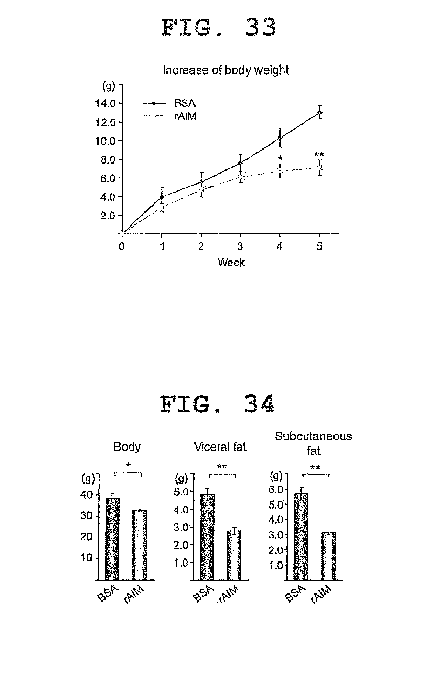

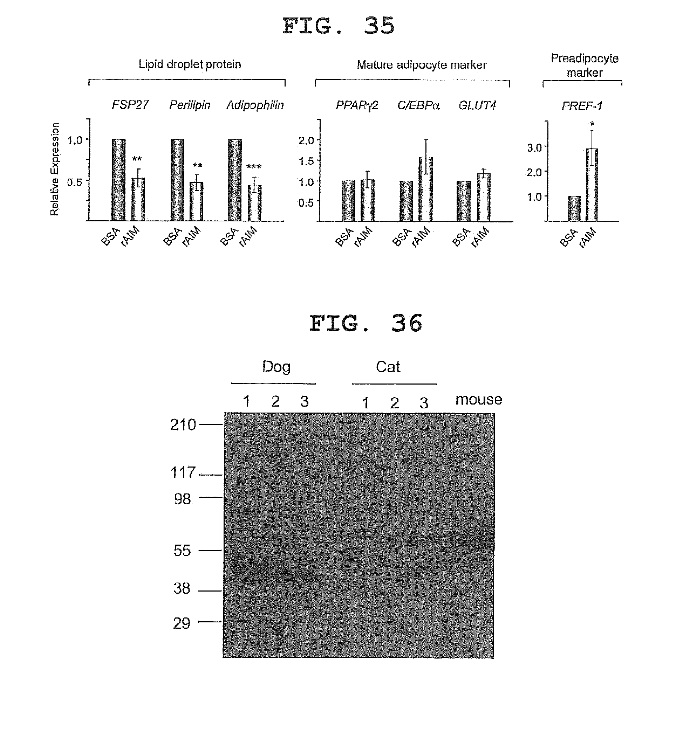

AIM^{+/+} mice (n=7) and AIM⁻¹⁻ mice (n=6) were fed with HFD for 12 weeks, and changes of visceral fat and subcutaneous fat amounts were measured. The results are shown in Fig. 32. The amount of increase in visceral fat (epididymal adipose tissue) and subcutaneous fat was greater in AIM^{-/-} mice.
Then, rAIM was intraperitoneally administered twice a week (n=6; 300 µg/injection/mouse) to AIM^{-/-} mice for 5 weeks while feeding with HFD, and the body weight and adipose tissue amount were measured. The results are shown in Figs. 33 and 34. The rAIM administration group showed a significantly small increase in all of the body weight, visceral fat and subcutaneous fat, as compared to the control administered with bovine serum albumin (n=5; 300 µg/injection/mouse).
The mRNA level of adipocyte marker, preadipocyte marker, and lipid droplet formation-related genes in the RNA extracted from visceral fat tissue of the mice of the rAIM administration group and BSA administration group was measured by QPCR. The results are shown in Fig. 35. The measurement values were standardized with the measurement values of GAPDH, and shown by an expression level relative to that of the adipose tissue administered with BSA.
In the rAIM administration group, the mRNA level of FSP27, Perilipin, and Adipophilin in visceral fat was also low. On the other hand, the mRNA level of PREF-1 increased by the administration of rAIM, but mRNA of PPARγ2, C/EBPα, GLUT4 did not show difference between the rAIM administration group and BSA administration group.

### Reference Example 8. Expression of AIM in dog and cat

AIM protein in the sera of dog, cat and mouse was detected by Western blotting (reducing condition) using an anti-AIM antibody (SA-1).
The results are shown in Fig. 36. As shown in the Figure, AIM protein was detected in all animals. The difference in the molecular weight between species is considered to be attributable to the modification of sugar chain and the like.

### Example 5. Preparation of diagnostic anti-AIM antibody

An anti-AIM antibody usable for diagnosis was prepared.

### <animal immunization>

As an antigen, human rAIM (2 mg/mL) was mixed with an equal amount of TiterMax Gold (G-1 Funakoshi) to give an emulsion. As an animal to be immunized, two 6-week-old female Jcl:Wistar rats (CLEA Japan, Inc.) were used and 50 µL was administered to the hind limb plantar region. After 2 weeks, similar administration was performed, and after 2 weeks or longer, and an antigen solution (50 µg) was administered to the plantar region to prepare for the cell fusion 3 days later.

### <myeloma cell>

As myeloma cell, mouse P3U1 was used and, as a growth culture, a medium prepared by adding glutamine and pyruvic acid to RPMI1640 (11875-119 GIBCO) and adding FBS (S1560 BWT Inc.) to 10% was used. As an antibiotic, an appropriate amount of penicillin or streptomycin was added.

### <cell fusion>

Under anesthesia, heart blood was drawn from a rat, popliteal lymph node was isolated aseptically, placed on a beaker with #200 mesh and a cell suspension was prepared by pushing with a silicon rod. The cells were washed with RPMI1640 by two times of centrifugation, and counted.
Myeloma cells in the logarithmic growth phase were collected by centrifugation, washed, prepared to 5:1 relative to the lymph cell, and subjected to mix centrifugation.
Cell fusion was performed using PEG1500 (783641 Roche). That is, 1 mL of PEG solution was reacted with cell pellets for 3 min, the reaction mixture was stepwisely diluted, and washed by centrifugation. The medium was added, and the mixture was added to 96 well plates (15 plates) by 200 µL, and cultured for 1 week. As the medium, a medium for myeloma cell added with HAT supplement (21060-017 GIBCO) and adjusted to FBS concentration of 15% was used.

### <collection of mouse ascites>

Cryopreserved cells were thawed, growth culture was performed, 1×10⁷ cells were administered to the abdominal cavity of nude mouse (BALB/cAJcl-nu/nu CLEA Japan, Inc.) intraperitoneally administered with 0.5 ml of pristine (42-002 COSMO BIO) not less than 1 week before, and 4 - 12 ml of ascites was obtained about 2 weeks later. Solid was removed by centrifugation treatment and the ascites was cryopreserved.

### <Electrophoresis analysis>

The ascites was thawed, treated with 5 µm filter, and the band of a monoclonal antibody contained in the ascites was confirmed by cellulose acetate membrane electrophoresis.
The electrophoresis conditions were 0.05 M Barbital Na Buffer pH 8.6 (020-13415 Wako Pure Chemical Industries, Ltd.), SELECA-V (ADVANTEC), 1 mA/cm, 25 min, fixing was performed, and 0.1% nigrosine (2% acetic acid) was used for staining.
Of the obtained clones, AIM-CL-6 and AIM-CL-7 were deposited according to the prescription of the Budapest Treaty. The deposit Nos. were NITE BP-1092 and NITE BP-1093, respectively.
Depository: Incorporated Administrative Agency National Institute of Technology and Evaluation Patent Microorganisms Depositary (NPMD: National Institute of Technology and Evaluation, Patent Microorganisms Depositary; 2-5-8 Kazusakamatari, Kisarazu-shi, Chiba, Japan)
Deposit date: May 2, 2011

### Example 6. Measurement of blood hAIM concentration of subjects who underwent medical checkup

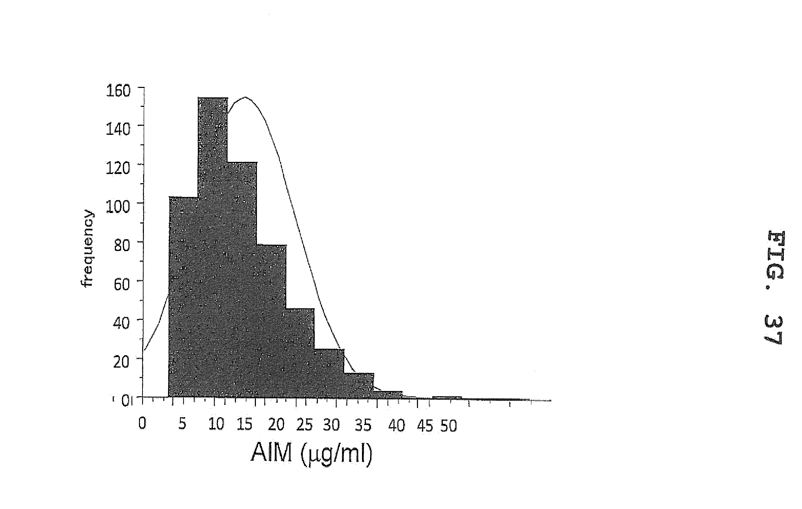

Using AIM-CL-6 and AIM-CL-7, obtained in Example 5, the blood hAIM concentration of about 550 subjects who underwent medical checkups was measured. To be specific, using AIM-CL-6 for trapping, AIM-CL-7 for detection, and serum (50 µL), the analysis was performed in duplicate. Human rAIM was sequentially diluted to set a standard curve, and the concentration was determined thereby.
Human rAIM was obtained by producing human AIM protein labeled with HA-tag in HEK293T cells, and column purifying the protein from the culture supernatant using an anti-HA antibody.
The results are shown in Fig. 37. Many healthy individuals showed a blood AIM concentration within the range of 5 µg/ml - 20 µg/ml.

### Example 7. Relationship between BMI and blood AIM concentration

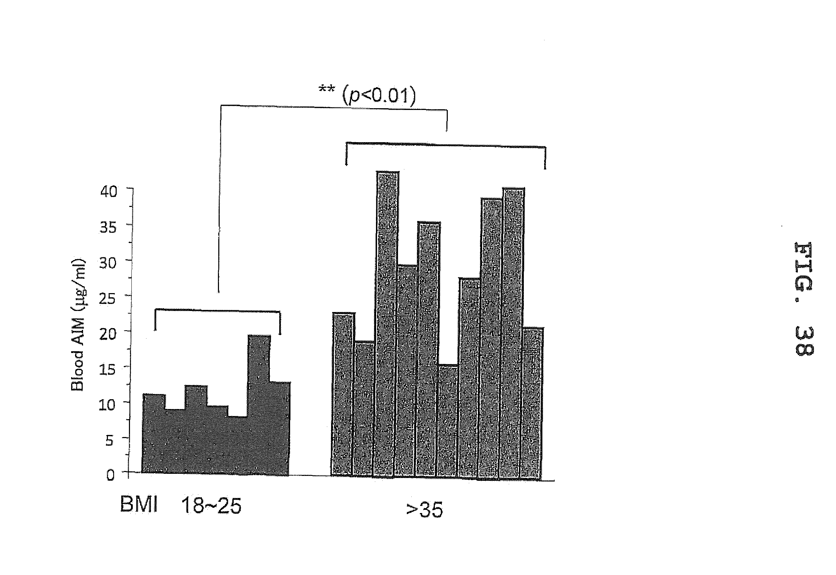

From blood donors (including foreigners), donors having BMI of 18 - 25 or not less than 35 were selected at random, and the blood AIM concentration was measured by a method similar to that in Fig. 6. Generally, a higher BMI is said to be associated with a higher risk of metabolic syndrome.
The results are shown in Fig. 38. The blood AIM concentration was significantly high in those having BMI of not less than 35, as compared to those having BMI of 18 - 25. This suggests that the measurement results of blood AIM concentration can be used for the diagnosis or detection of metabolic syndrome and related diseases thereof.

### [Sequence Listing Free Text]

SEQ ID NO: 1 is the amino acid sequence of human AIM.
SEQ ID NO: 2 is the DNA sequence of forward primer used for the PPARγ1 expression analysis by quantitative real-time PCR.
SEQ ID NO: 3 is the DNA sequence of reverse primer used for the PPARγ1 expression analysis by quantitative real-time PCR.
SEQ ID NO: 4 is the DNA sequence of forward primer used for the PPARγ2 expression analysis by quantitative real-time PCR.
SEQ ID NO: 5 is the DNA sequence of reverse primer used for the PPARγ2 expression analysis by quantitative real-times PCR.
SEQ ID NO: 6 is the DNA sequence of forward primer used for the C/EBPα expression analysis by quantitative real-time PCR.
SEQ ID NO: 7 is the DNA sequence of reverse primer used for the C/EBPα expression analysis by quantitative real-time PCR.
SEQ ID NO: 8 is the DNA sequence of forward primer used for the CD36 expression analysis by quantitative real-time PCR.
SEQ ID NO: 9 is the DNA sequence of reverse primer used for the CD36 expression analysis by quantitative real-time PCR.
SEQ ID NO: 10 is the DNA sequence of forward primer used for the GLUT4 expression analysis by quantitative real-time PCR.
SEQ ID NO: 11 is the DNA sequence of reverse primer used for the GLUT4 expression analysis by quantitative real-time PCR.
SEQ ID NO: 12 is the DNA sequence of forward primer used for the Fsp27 expression analysis by quantitative real-time PCR.
SEQ ID NO: 13 is the DNA sequence of reverse primer used for the Fsp27 expression analysis by quantitative real-time PCR.
SEQ ID NO: 14 is the DNA sequence of forward primer used for the Perilipin expression analysis by quantitative real-time PCR.
SEQ ID NO: 15 is the DNA sequence of reverse primer used for the Perilipin expression analysis by quantitative real-time PCR.
SEQ ID NO: 16 is the DNA sequence of forward primer used for the Adipophilin expression analysis by quantitative real-time PCR.
SEQ ID NO: 17 is the DNA sequence of reverse primer used for the Adipophilin expression analysis by quantitative renal-time PCR.
SEQ ID NO: 18 is the DNA sequence of forward primer used for the GAPDH expression analysis by quantitative real-time PCR.
SEQ ID NO: 19 is the DNA sequence of reverse primer used for the GAPDH expression analysis by quantitative real-time PCR,
SEQ ID NO: 20 is the DNA sequence of forward primer used for the PREF1 expression analysis by quantitative real-time PCR.
SEQ ID NO: 21 is the DNA sequence of reverse primer used for the PREF1 expression analysis by quantitative real-time PCR.
SEQ ID NO: 22 is the amino acid sequence of chimpanzee AIM.
SEQ ID NO: 23 is the amino acid sequence of dog AIM.
SEQ ID NO: 24 is the amino acid sequence of mouse AIM.
SEQ ID NO: 25 is the amino acid sequence of rat AIM.

## Claims

1. A method for the diagnosis or detection of an AIM-related disease, comprising
a step of measuring an AIM concentration in a sample collected from a subject,
a step of comparing the aforementioned AIM concentration and an AIM concentration in a sample of a healthy subject.

2. The method according to claim 1, wherein the sample is a body fluid, an organ or a tissue.

3. The method according to claim 2, wherein the body fluid is blood.

4. The method according to claim 2, wherein the tissue is an adipose tissue.

5. The method according to any one of claims 1 to 4, wherein the step of measuring an AIM concentration is performed by a method selected from the group consisting of an immunoassay, an agglutination method, turbidimetry, a Western blotting method and a surface plasmon resonance method.

6. The method according to claim 5, wherein the immunoassay is selected from the group consisting of enzyme immunoassay (EIA or ELISA), radio immunoassay (RIA), fluorescence immunoassay (FIA), fluorescence polarization immunoassay (FPIA) and chemical luminescence immunoassay (CLIA), each using an anti-AIM antibody.

7. The method according to claim 6, wherein AIM-CL-6 (deposit number: NITE BP-1092) or AIM-CL-7 (deposit number: NITE BP 1093) is used as the anti-AIM antibody.

8. The method according to any one of claims 1 to 7, wherein the AIM-related disease is at least one disease selected from the group consisting of metabolic syndrome or a related disease thereof, cancer, infectious disease, degenerative disease of the brain and chronic inflammatory disease.

9. The method according to claim 8, wherein the AIM-related disease is metabolic syndrome or a related disease thereof, and
when, in the step of comparing the AIM concentration and the AIM concentration in a sample of a healthy subject, the AIM concentration in the sample of the subject is significantly higher than that in the sample of the healthy subject, the subject is judged to have been affected or have a high risk of being affected with metabolic syndrome or a related disease thereof.

10. The method according to claim 9, wherein the metabolic syndrome or a related disease thereof is at least one selected from the group consisting of metabolic syndrome, obesity, insulin resistance, diabetes, hyperlipemia, hypertension, arteriosclerotic disease, cerebrovascular disorder, ischemic heart disease, heart failure, dementia, cerebral apoplexy, neurosis, renal disease, abnormal secretion of adipocytokine and abnormal amount of blood free fatty acid.

11. The method according to any one of claims 1 to 10, wherein the subject is a human.

12. The method according to any one of claims 1 to 10, wherein the subject is a non-human mammal or bird.

13. The method according to claim 12, wherein the subject is a dog or cat.

14. A diagnostic or detection kit for AIM-related disease, comprising an anti-AIM antibody.

15. The kit according to claim 14, wherein the AIM-related disease is at least one selected from the group consisting of metabolic syndrome, cancer, infectious disease, degenerative disease of the brain and chronic inflammatory disease.

16. The kit according to claim 15, wherein the metabolic syndrome or related diseases thereof is at least one selected from the group consisting of metabolic syndrome, obesity, insulin resistance, diabetes, hyperlipidemia, hypertension, arteriosclerotic disease, cerebrovascular disorder, ischemic heart disease, heart failure, dementia, cerebral apoplexy, neurosis, renal disease, secretion abnormality of adipocytokine, and abnormality amount of blood free fatty acid.

17. The kit according to any one of claims 14 to 16, wherein the anti-AIM antibody is AIM-CL-6 (deposit number: NITE BP-1092) or AIM-CL-7 (deposit number: NITE BP 1093).

18. The method according to any one of claims 14 to 16, wherein the AIM antibody is a fragment containing a functional domain and a conserved region of the AIM protein.

19. A method of using CD36, which is for the diagnosis or detection of an AIM-related disease.

20. A method of using CD36, which is for the production of a diagnostic drug or a test drug for an AIM-related disease.

21. An anti-AIM antibody which is AIM-CL-6 (deposit number: NITE BP-1092) or AIM-CL-7 (deposit number: NITE BP 1093).


SEQUENCE LISTING

<110>  Miyazaki, Toru

<120>  Diagnostic Method for AIM-related Diseases

<130>  T0529ACP0009

<160>  25

<170>  PatentIn version 3.1

<210>  1
<211>  347
<212>  PRT
<213>  Homo sapiens

<220>
<221>  misc_feature
<223>  amino acid sequence of human AIM protein

<400>  1

Met Ala Leu Leu Phe Ser Leu Ile Leu Ala Ile Cys Thr Arg Pro Gly
1               5                   10                  15

Phe Leu Ala Ser Pro Ser Gly Val Arg Leu Val Gly Gly Leu His Arg
            20                  25                  30

Cys Glu Gly Arg Val Glu Val Glu Gln Lys Gly Gln Trp Gly Thr Val
        35                  40                  45

Cys Asp Asp Gly Trp Asp Ile Lys Asp Val Ala Val Leu Cys Arg Glu
    50                  55                  60

Leu Gly Cys Gly Ala Ala Ser Gly Thr Pro Ser Gly Ile Leu Tyr Glu
65                  70                  75                  80

Pro Pro Ala Glu Lys Glu Gln Lys Val Leu Ile Gln Ser Val Ser Cys
                85                  90                  95

Thr Gly Thr Glu Asp Thr Leu Ala Gln Cys Glu Gln Glu Glu Val Tyr
            100                 105                 110

Asp Cys Ser His Asp Glu Asp Ala Gly Ala Ser Cys Glu Asn Pro Glu
        115                 120                 125

Ser Ser Phe Ser Pro Val Pro Glu Gly Val Arg Leu Ala Asp Gly Pro
    130                 135                 140

Gly His Cys Lys Gly Arg Val Glu Val Lys His Gln Asn Gln Trp Tyr
145                 150                 155                 160

Thr Val Cys Gln Thr Gly Trp Ser Leu Arg Ala Ala Lys Val Val Cys

165                 170                 175

Arg Gln Leu Gly Cys Gly Arg Ala Val Leu Thr Gln Lys Arg Cys Asn
            180                 185                 190

Lys His Ala Tyr Gly Arg Lys Pro Ile Trp Leu Ser Gln Met Ser Cys
        195                 200                 205

Ser Gly Arg Glu Ala Thr Leu Gln Asp Cys Pro Ser Gly Pro Trp Gly
    210                 215                 220

Lys Asn Thr Cys Asn His Asp Glu Asp Thr Trp Val Glu Cys Glu Asp
225                 230                 235                 240

Pro Phe Asp Leu Arg Leu Val Gly Gly Asp Asn Leu Cys Ser Gly Arg
                245                 250                 255

Leu Glu Val Leu His Lys Gly Val Trp Gly Ser Val Cys Asp Asp Asn
            260                 265                 270

Trp Gly Glu Lys Glu Asp Gln Val Val Cys Lys Gln Leu Gly Cys Gly
        275                 280                 285

Lys Ser Leu Ser Pro Ser Phe Arg Asp Arg Lys Cys Tyr Gly Pro Gly
    290                 295                 300

Val Gly Arg Ile Trp Leu Asp Asn Val Arg Cys Ser Gly Glu Glu Gln
305                 310                 315                 320

Ser Leu Glu Gln Cys Gln His Arg Phe Trp Gly Phe His Asp Cys Thr
                325                 330                 335

His Gln Glu Asp Val Ala Val Ile Cys Ser Gly
            340                 345

<210>  2
<211>  23
<212>  DNA
<213>  Artificial

<220>
<223>  DNA sequence of forward primer used in expression analysis of PPA
       R gamma 1 by quantitative real-time PCR

<400>  2
caagaatacc aaagtgcgat caa                                              23

<210>  3
<211>  26
<212>  DNA
<213>  Artificial

<220>
<223>  DNA sequence of reverse primer used in expression analysis of PPA
       R gamma 1 by quantitative real-time PCR

<400>  3
gagctgggtc ttttcagaat aataag                                          26


<210>  4
<211>  21
<212>  DNA
<213>  Artificial

<220>
<223>  DNA sequence of forward primer used in expression analysis of PPA
       R gamma 2 by quantitative real-time PCR

<400>  4
ccagagcatg gtgccttcgc t                                               21


<210>  5
<211>  21
<212>  DNA
<213>  Artificial

<220>
<223>  DNA sequence of reverse primer used in expression analysis of PPA
       R gamma 2 by quantitative real-time PCR

<400>  5
cagcaaccat tgggtcagct c                                               21


<210>  6
<211>  20
<212>  DNA
<213>  Artificial

<220>
<223>  DNA sequence of forward primer used in expression analysis of C/E
       BP alpha by quantitative real-time PCR

<400>  6
agcaacgagt accgggtacg                                                 20


<210>  7
<211>  21
<212>  DNA
<213>  Artificial

<220>
<223>  DNA sequence of reverse primer used in expression analysis of C/E
       BP alpha by quantitative real-time PCR

<400>  7
tgtttggctt tatctcggct c                                               21


<210>  8
<211>  27
<212>  DNA
<213>  Artificial

<220>
<223>  DNA sequence of forward primer used in expression analysis of CD3
       6 by quantitative real-time PCR

<400>  8
ttgtacctat actgtggcta aatgaga                                          27


<210>  9
<211>  24
<212>  DNA
<213>  Artificial

<220>
<223>  DNA sequence of reverse primer used in expression analysis of CD3
       6 by quantitative real-time PCR

<400>  9
cttgtgtttt gaacatttct gctt                                             24


<210>  10
<211>  20
<212>  DNA
<213>  Artificial

<220>
<223>  DNA sequence of forward primer used in expression analysis of GLU
       T4 by quantitative real-time PCR

<400>  10
gacggacact ccatctgttg                                                  20


<210>  11
<211>  19
<212>  DNA
<213>  Artificial

<220>
<223>  DNA sequence of reverse primer used in expression analysis of GLU
       T4 by quantitative real-time PCR

<400>  11
gccacgatgg agacatagc                                                   19


<210>  12
<211>  24
<212>  DNA
<213>  Artificial

<220>
<223>  DNA sequence of forward primer used in expression analysis of Fsp
       27 by quantitative real-time PCR

<400>  12
ctggaggaag atggcacaat cgtg                                             24


<210>  13
<211>  25
<212>  DNA
<213>  Artificial

<220>
<223>  DNA sequence of reverse primer used in expression analysis of Fsp
      27 by quantitative real-time PCR

<400>  13
cagccaataa agtcctgagg gttca                                          25


<210>  14
<211>  17
<212>  DNA
<213>  Artificial

<220>
<223>  DNA sequence of forward primer used in expression analysis of Per
      ilipin by quantitative real-time PCR

<400>  14
tgctggatgg agacctc                                                   17


<210>  15
<211>  17
<212>  DNA
<213>  Artificial

<220>
<223>  DNA sequence of reverse primer used in expression analysis of Per
      ilipin by quantitative real-time PCR

<400>  15
accggctcca tgctcca                                                   17


<210>  16
<211>  21
<212>  DNA
<213>  Artificial

<220>
<223>  DNA sequence of forward primer used in expression analysis of Adi
      pophilin by quantitative real-time PCR

<400>  16
aagcatcggc tacgacgaca c                                              21


<210>  17
<211>  24
<212>  DNA
<213>  Artificial

<220>
<223>  DNA sequence of reverse primer used in expression analysis of Adi
      pophilin by quantitative real-time PCR

<400>  17
ggacagtctg gcatgtagtc tgga                                           24


<210>  18
<211>  20
<212>  DNA
<213>  Artificial

<220>
<223>  DNA sequence of forward primer used in expression analysis of GAP
       DH by quantitative real-time PCR

<400>  18
aactttggca ttgtggaagg                                                   20


<210>  19
<211>  20
<212>  DNA
<213>  Artificial

<220>
<223>  DNA sequence of reverse primer used in expression analysis of GAP
       DH by quantitative real-time PCR

<400>  19
ggatgcaggg atgatgttct                                                   20


<210>  20
<211>  20
<212>  DNA
<213>  Artificial

<220>
<223>  DNA sequence of forward primer used in expression analysis of PRE
       F1 by quantitative real-time PCR

<400>  20
cgggaaattc tgcgaaatag                                                   20


<210>  21
<211>  20
<212>  DNA
<213>  Artificial

<220>
<223>  DNA sequence of reverse primer used in expression analysis of PRE
       F1 by quantitative real-time PCR

<400>  21
tgtgcaggag cattcgtact                                                   20


<210>  22
<211>  347
<212>  PRT
<213>  Pan troglodytes

<220>
<221>  misc_feature
<223>  amino acid sequence of chimpanzee AIM protein


<400>  22

Met Ala Leu Leu Phe Ser Leu Ile Leu Ala Ile Cys Thr Arg Pro Gly
1               5                   10                  15


Phe Leu Ala Ser Pro Ser Gly Val Arg Leu Val Gly Gly Leu His Arg
            20                  25                  30

Cys Glu Gly Arg Val Glu Val Glu Gln Lys Gly Gln Trp Gly Thr Val
        35                  40                  45

Cys Asp Asp Gly Trp Asp Ile Lys Asp Val Ala Val Val Cys Arg Glu
    50                  55                  60

Leu Gly Cys Gly Ala Ala Ser Gly Thr Pro Ser Gly Ile Leu Tyr Glu
65                  70                  75                  80

Pro Pro Ala Glu Lys Glu Gln Lys Val Leu Ile Gln Ser Val Ser Cys
                85                  90                  95

Thr Gly Thr Glu Asp Thr Leu Ala Gln Cys Glu Gln Glu Glu Val Tyr
            100                 105                 110

Asp Cys Ser His Asp Glu Asp Ala Gly Ala Ser Cys Glu Asn Pro Glu
        115                 120                 125

Ser Ser Phe Ser Pro Val Pro Glu Gly Val Arg Leu Ala Asp Gly Pro
    130                 135                 140

Gly His Cys Lys Gly Arg Val Glu Val Lys His Gln Asn Gln Trp Tyr
145                 150                 155                 160

Thr Val Cys Gln Thr Gly Trp Ser Leu Arg Ala Ala Lys Val Val Cys
                165                 170                 175

Arg Gln Leu Gly Cys Gly Arg Ala Val Leu Thr Gln Lys Arg Cys Asn
            180                 185                 190

Lys His Ala Tyr Gly Arg Lys Pro Ile Trp Leu Ser His Met Ser Cys
        195                 200                 205

Ser Gly Arg Glu Ala Thr Leu Gln Asp Cys Pro Ser Gly Pro Trp Gly
    210                 215                 220

Lys Asn Thr Cys Asn His Asp Glu Asp Thr Trp Val Glu Cys Glu Asp
225                 230                 235                 240

Pro Phe Asp Leu Arg Leu Val Gly Gly Asp Asn Leu Cys Cys Gly Arg
                245                 250                 255

Leu Glu Val Leu His Lys Gly Val Trp Gly Ser Val Cys Asp Asp Asn
            260                 265                 270

Trp Gly Glu Lys Glu Asp Gln Val Val Cys Lys Gln Leu Gly Cys Gly
        275                 280                 285

Lys Ser Leu Ser Pro Ser Phe Arg Asn Arg Lys Cys Tyr Gly Pro Gly
    290                 295                 300

Val Gly Arg Ile Trp Leu Asp Asn Val Arg Cys Ser Gly Glu Glu Gln
305                 310                 315                 320

Ser Leu Glu Gln Cys Gln His Arg Phe Trp Gly Phe His Asp Cys Thr
                325                 330                 335

His Gln Glu Asp Val Ala Val Phe Cys Ser Val
            340                 345

<210>  23
<211>  340
<212>  PRT
<213>  Canis familiaris

<220>
<221>  misc_feature
<223>  amino acid sequence of dog AIM protein

<400>  23

Met Ala Leu Leu Phe Ser Leu Ile Leu Ala Ile Cys Thr Gly Pro Gly
1               5                   10                  15

Phe Leu Glu Ser Ser Ser Arg Val Arg Leu Val Gly Gly Asp His Arg
            20                  25                  30

Cys Glu Gly Arg Val Glu Val Gln Arg Tyr Gly Glu Trp Gly Thr Ile
        35                  40                  45

Cys Asp Asp Gly Trp Asp Ile Lys Asp Val Ala Val Val Cys Arg Glu
    50                  55                  60

Leu Asp Cys Gly Val Ala Lys Arg Ala Leu Ser Gly Thr Leu Phe Gly
65                  70                  75                  80

Pro Pro Thr Gln Gln Gln Gln Lys Ile Phe Ile Gln Gln Val Lys Cys
                85                  90                  95

His Gly Met Glu Glu Asn Leu Ser Gln Cys Glu Glu Glu Asp Ala Phe
            100                 105                 110

Asp Cys Thr His Asp Glu Asp Ala Gly Val Val Cys Gly Phe Pro Glu
        115                 120                 125

Asn Val Arg Leu Val Asp Gly His Lys Arg Cys Gln Gly Arg Val Glu
    130                 135                 140

Val Lys Gln Gln Gly Gln Trp Gly Thr Val Cys Lys Gly Ser Trp Asn
145                 150                 155                 160

Phe Ala Ala Ala Lys Val Val Cys Gln Gln Val Gly Cys Gly Arg Ala
                165                 170                 175

Ile Leu Thr Arg Lys Cys Cys Asn Lys Ala Thr Gln Gly Gln Gly Pro
            180                 185                 190

Ile Trp Pro Arg Lys Val Ser Cys Ser Gly Lys Glu Ile Ser Leu Gln
        195                 200                 205

Asp Cys Pro Ser Glu Val Trp Glu Lys Asn Asn Cys Thr His Asp Glu
    210                 215                 220

Asp Met Trp Val Glu Cys Glu Asp Pro Phe Asp Leu Lys Leu Val Gly
225                 230                 235                 240

Gly Asp Ser Pro Cys Ala Gly Arg Leu Glu Val Leu His Lys Gly Glu
                245                 250                 255

Trp Gly Thr Val Cys Asp Asp Asp Trp Gly Glu Met Ala Asp Gln Val
            260                 265                 270

Val Cys Arg Gln Leu Gly Cys Gly Ala Ser Leu Ser Pro Ser Val Lys
        275                 280                 285

Phe Arg Arg Arg Phe Gly Pro Gly Val Gly Arg Ile Trp Leu Asp Asp
    290                 295                 300

Val Ala Cys Ser Gly Asn Glu Arg Ser Leu Glu Gln Cys Arg His Arg
305                 310                 315                 320

Phe Trp Gly His His Asn Cys Lys His Thr Glu Asp Val Ala Val Val
                325                 330                 335

Cys Ala Glu Gln
            340

<210>  24
<211>  352
<212>  PRT
<213>  Mus musculus

<220>
<221>  misc_feature
<223>  amino acid sequence of mouse AIM protein

<400>  24

Met Ala Pro Leu Phe Asn Leu Met Leu Ala Ile Leu Ser Ile Phe Val

1               5                   10                  15

Gly Ser Cys Phe Ser Glu Ser Pro Thr Lys Val Gln Leu Val Gly Gly
            20                  25                  30

Ala His Arg Cys Glu Gly Arg Val Glu Val Glu His Asn Gly Gln Trp
        35                  40                  45

Gly Thr Val Cys Asp Asp Gly Trp Asp Arg Arg Asp Val Ala Val Val
    50                  55                  60

Cys Arg Glu Leu Asn Cys Gly Ala Val Ile Gln Thr Pro Arg Gly Ala
65                  70                  75                  80

Ser Tyr Gln Pro Pro Ala Ser Glu Gln Arg Val Leu Ile Gln Gly Val
                85                  90                  95

Asp Cys Asn Gly Thr Glu Asp Thr Leu Ala Gln Cys Glu Leu Asn Tyr
            100                 105                 110

Tyr Val Phe Asp Cys Ser His Glu Glu Asp Ala Gly Ala Gln Cys Glu
        115                 120                 125

Asn Pro Asp Ser Asp Leu Leu Phe Ile Pro Glu Asp Val Arg Leu Val
    130                 135                 140

Asp Gly Pro Gly His Cys Gln Gly Arg Val Glu Val Leu His Gln Ser
145                 150                 155                 160

Gln Trp Ser Thr Val Cys Lys Ala Gly Trp Asn Leu Gln Val Ser Lys
                165                 170                 175

Val Val Cys Arg Gln Leu Gly Cys Gly Arg Ala Leu Leu Thr Tyr Gly
            180                 185                 190

Ser Cys Asn Lys Asn Thr Gln Gly Lys Gly Pro Ile Trp Met Gly Lys
        195                 200                 205

Met Ser Cys Ser Gly Gln Glu Ala Asn Leu Arg Ser Cys Leu Leu Ser
    210                 215                 220

Arg Leu Glu Asn Asn Cys Thr His Gly Glu Asp Thr Trp Met Glu Cys
225                 230                 235                 240

Glu Asp Pro Phe Glu Leu Lys Leu Val Gly Gly Asp Thr Pro Cys Ser
                245                 250                 255

Gly Arg Leu Glu Val Leu His Lys Gly Ser Trp Gly Ser Val Cys Asp
            260                 265                 270

Asp Asn Trp Gly Glu Lys Glu Asp Gln Val Val Cys Lys Gln Leu Gly
        275                 280                 285

Cys Gly Lys Ser Leu His Pro Ser Pro Lys Thr Arg Lys Ile Tyr Gly
    290                 295                 300

Pro Gly Ala Gly Arg Ile Trp Leu Asp Asp Val Asn Cys Ser Gly Lys
305                 310                 315                 320

Glu Gln Ser Leu Glu Phe Cys Arg His Arg Leu Trp Gly Tyr His Asp
                325                 330                 335

Cys Thr His Lys Glu Asp Val Glu Val Ile Cys Thr Asp Phe Asp Val
            340                 345                 350

<210>  25
<211>  346
<212>  PRT
<213>  Rattus norvegicus

<220>
<221>  misc_feature
<223>  amino acid sequence of rat AIM protein

<400>  25

Met Ala Leu Leu Phe Asn Leu Ile Leu Ala Leu Leu Thr Ile Phe Val
1               5                   10                  15

Gly Leu Cys Phe Ser Glu Ser Pro Thr Arg Val Arg Leu Val Gly Gly
            20                  25                  30

Ala His Arg Cys Glu Gly Arg Val Glu Val Lys His Asn Gly Gln Trp
        35                  40                  45

Gly Thr Val Cys Asp Asp Gly Trp Asp Leu Ile Asp Val Ser Val Val
    50                  55                  60

Cys Arg Glu Leu Asn Cys Gly Ala Ala Lys Lys Thr Pro Ser Gly Ala
65                  70                  75                  80

Ser Tyr His Pro Pro Ala Ser Glu Gly Gln Ser Val Leu Ile Gln Gly
                85                  90                  95

Val Glu Cys Ser Gly Ala Glu Asp Met Leu Ala Gln Cys Thr Leu Asn
            100                 105                 110

Tyr Asp Val Phe Asp Cys Ser His Ala Glu Asp Ala Gly Val Gln Cys
        115                 120                 125

Glu Asn Pro Asp Asn Pro Glu Asn Val Arg Leu Val Glu Gly Leu Gly
    130                 135                 140

Arg Cys Gln Gly Arg Leu Glu Val Phe Tyr Gln Gly Gln Trp Ser Thr
145                 150                 155                 160

Val Cys Lys Ala Gly Trp Asn Leu Gln Ala Ser Lys Val Val Cys Arg
                165                 170                 175

Gln Leu Gly Cys Gly Arg Ala Leu Leu Ala His Arg Cys Cys Asn Lys
            180                 185                 190

Asn Thr Gln Gly Lys Gly Pro Ile Trp Met Ser Lys Met Ser Cys Ser
        195                 200                 205

Gly Arg Glu Ala Asn Leu Gln Asp Cys Pro Phe Ser Pro Leu Glu Ser
    210                 215                 220

Asn Cys Thr His Gly Glu Asp Thr Trp Met Glu Cys Glu Asp Pro Phe
225                 230                 235                 240

Glu Leu Arg Leu Val Gly Gly Asp Thr Thr Cys Ser Gly Arg Leu Glu
                245                 250                 255

Val Leu His Lys Gly Ser Trp Gly Ser Val Cys Asp Asp Ser Trp Gly
            260                 265                 270

Glu Lys Glu Asp Gln Val Val Cys Lys Gln Leu Gly Cys Gly Lys Ser
        275                 280                 285

Leu Phe Pro Ser Pro Lys Ala Arg Gln Ser Phe Gly Pro Gly Thr Gly
    290                 295                 300

Arg Ile Trp Leu Asp Asp Val Ser Cys Ser Gly Lys Glu Glu Ser Leu
305                 310                 315                 320

Glu Leu Cys Arg His Arg Leu Trp Gly Tyr His Asp Cys Thr His Lys
                325                 330                 335

Glu Asp Val Gly Val Ile Cys Ser Glu Leu
            340                 345